# EUROPEAN PATENT APPLICATION

(11) **EP 1 640 369 A1**
(43) Date of publication of application: **29.03.2006**
(21) Application number: 04746718.8
(22) Date of filing: 23.06.2004
(51) Int. Cl.: C07D 277/46, C07D 277/40, C07D 417/12, C07D 417/04, A61K 31/426, A61K 31/454, A61P 9/10, A61P 9/12, A61P 11/02, A61P 11/06, A61P 13/12, A61P 25/02, A61P 25/18, A61P 25/22, A61P 25/24, A61P 25/28, A61P 27/02, A61P 27/06, A61P 37/08, A61P 43/00

(54) **THIAZOLIMINE COMPOUND AND OXAZOLIMINE COMPOUND**

(30) Priority: 27.06.2003 JP 2003184321
(71) Applicant: Dainippon Sumitomo Pharma Co., Ltd., Osaka 541-8524 (JP)
(72) Inventor: Yamaguchi, H., Sumitomo Pharmaceuticals Co.,Ltd., Osaka 5540022 (JP)
(74) Representative: Duckett, Anthony Joseph
(86) International application number: PCT/JP2004/009249
(87) International publication number: WO 2005/000825

(57) **Abstract**

A compound represented by the formula (1): 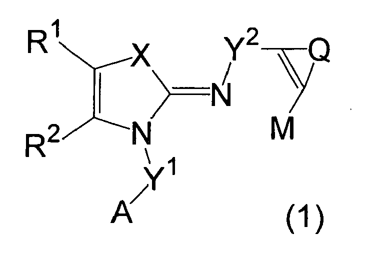 (wherein X represents sulfur or oxygen; R¹ and R² each represents a group represented by the formula -Y³-Z, etc.; Y³ represents a single bond or (un)substituted alkylene; Y¹ and Y² each represents (un)substituted alkylene; Z represents hydrogen, an (un)saturated monocyclic heterocyclic group, etc.; M represents carboxy, etc.; Q represents o-phenylene, etc.; and A represents an (un)saturated monocyclic hydrocarbon group, etc.), a prodrug thereof, or a pharmaceutically acceptable salt of either. They are compounds having chymase inhibitory activity and useful as a therapeutic agent for hypertension, cardiac failure, etc.

## Description

### TECHNICAL FIELD

The present invention relates to novel thiazolimine or oxazolimine compounds, prodrugs thereof or pharmaceutically acceptable salts of the compounds or prodrugs.

Chymase is one of intracellular enzymes found in granules secreted by mast cells and is a member of the subfamily of chymotrypsin-like serine proteases. Chymase has, for example, the following biological actions: when released outside a cell, chymase binds to the surrounding extracellular matrix rapidly, severs extracellular substrates such as type IV collagen and fibronectin, enhances the vasopermeability together with histamine, increases the action of histamine, produces histamine-free peptides from serum albumin, limitedly degrades IgG, forms leukocyte migration factor, and activates a precursor of interleukin-1β, one of inflammatory cytokines.

It has been reported that chymase has an action of causing the activation of mast cells themselves. On the other hand, it has been revealed that chymase participates in the conversion of angiotensin I to angiotensin II. It has been considered that angiotensin converting enzyme (hereinafter abbreviated as ACE) affects the production of angiotensin II, but it has recently been revealed that ACE affects only approximately 10 to 15% of the production of angiotensin II in human heart and that human-type chymase affects 80% or more of the production.

In addition, it has also been found that chymase acts on many physiologically active substances as substrates, such as endothelin production process, substance P, vasoactive intestinal polypeptide (VIP), apoprotein B and the like. Moreover, it has also been revealed that chymase participates also in the activation of other intracellular proteases such as collagenase. Furthermore, it has also been revealed that chymase acts also on ApoA-I as substrate and hence has inhibitory effect on the reverse phase system of cholesterol. As to the distribution of chymase, it has been confirmed that mast cells are present outside the blood vessels of heart and that chymase activity is present in mast cells and interstitial tissue while binding to an extracellular substrate. Chymase is distributed in a high proportion also in skin, lungs, liver and renal cortex besides heart. Chymase having such various physiological activities is known to be responsible for a variety of pathosis and is known to be responsible for, for example, myocardiac infarction, cardiac failure, restenosis after PTCA (percutaneous transluminal coronary angioplasty), hypertension, allergic diseases and organ fibrosis.

Therefore, it is conjectured that an activity inhibitor for chymase is useful as a therapeutic agent for cardiovascular disorder, a therapeutic agent for arteriosclerosis, an anti-inflammatory agent, an antiallergic agent or the like. More specifically, a compound having inhibitory effect on chymase is useful as a therapeutic agent for a disease whose pathosis is considered improvable by this effect, such as the following diseases in which angiotensin II, endothelin or the like is involved: hypertension, cardiac failure, ischemic peripheral circulatory disturbance, myocardial ischemia, venous malfunction, cardiac failure advance after myocardiac infarction, diabetic nephropathy, nephritis, arteriosclerosis, hyperaldosteronism, scleroderma, glomerulosclerosis, renal failure, central nervous system diseases, Alzheimer's disease, hypomnesia, depression, sensory functional disorders including amnesia and senile dementia, anxiety and tension, unpleasant mental condition, glaucoma, ocular hypertension, restenosis after PTCA, asthma, rhinitis, COPD (chronic obstructive pulmonary disease), allergic diseases such as atopic dermatitis, and the like.

### BACKGROUND ART

As the compound having inhibitory effect on chymase, there may be exemplified benzimidazole derivatives (see International Publication No. WO00/03997 pamphlet), pyrimidone derivatives (see International Publication No. WO99/41277 pamphlet) and quinazolinone derivatives (see International Publication No. WO00/10982 pamphlet). These compounds, however, are different in structure from the compounds of the present invention.

As thiazolimine compounds and oxazolimine compounds, there are known, for example, the compounds disclosed in International Publication No. WO02/02542 pamphlet and International Publication No. WO92/15564 pamphlet. The compounds of the present invention are different from these compounds in structure because as shown in formula (1), they have a substituent with a specified partial structure on the nitrogen atom of an imino group.

### DISCLOSURE OF THE INVENTION

A problem to be solved by the present invention is to provide a compound that has chymase inhibitory activity and is useful as a therapeutic agent for the above-exemplified diseases.

The present inventors earnestly investigated in order to solve the above problem, and consequently found that a compound represented by formula (1), a prodrug thereof, or a pharmaceutically acceptable salt of either (if necessary, they are hereinafter abbreviated as "compound of the present invention" in some cases) has an excellent inhibitory effect on chymase. That is, the present invention relates to the following.
[1] A compound represented by formula (1): 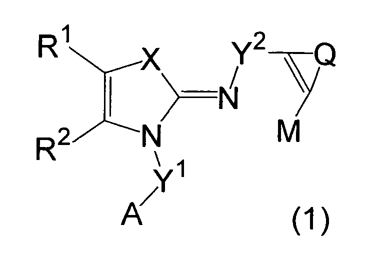 wherein X is a sulfur atom or an oxygen atom;
   R¹ and R² are independently a group represented by the formula: -Y³-Z, or R¹ and R², when taken together, represent a substituted or unsubstituted alkylene group (the -CH₂- groups of the alkylene group may be replaced by one or more substituents which may be the same or different and are selected from groups represented by the formulas: -O-, -S(O)ₙ-, -N(R¹¹) - and -C(=O)-);
   Y³ is a single bond or a substituted or unsubstituted alkylene group (the -CH₂- groups of the alkylene group may be replaced by one or more substituents which may be the same or different and are selected from groups represented by the formulas: -O-, -S(O)ₙ-, -N (R¹¹) - and -C (=O) -, substituted or unsubstituted benzene rings, and substituted or unsubstituted cycloalkane rings);
   Y¹ and Y² are independently a substituted or unsubstituted alkylene group (the -CH₂- groups of the alkylene group may be replaced by one or more substituents which may be the same or different and are selected from groups represented by the formulas: -O-, -S(O)ₙ-, -N(R¹¹)- and -C(=O)-, substituted or unsubstituted benzene rings, and substituted or unsubstituted cycloalkane rings, provided that the end of the alkylene group directly bonded to each nitrogen atom in formula (1) is not a group represented by the formula: -N(R¹¹)-);
   the -CH₂- groups in a cycloalkane ring in the case of the cycloalkane ring being present in any of Y¹, Y² and Y³ may be replaced by one or more substituents which may be the same or different and are selected from groups represented by the formulas: -O-, -S(O)ₙ-, -N(R¹¹)- and -C (=O) -;
   any adjacent two carbon atoms of an alkylene group may form a double bond or a triple bond in the case of the alkylene group being present as any of Y¹, Y² and Y³ or in the case of R¹ and R² being taken together to represent the alkylene group;
   Z is a saturated or unsaturated monocyclic hydrocarbon ring group, a saturated or unsaturated polycyclic hydrocarbon ring group, a saturated or unsaturated monocyclic heterocyclic group, or a saturated or unsaturated polycyclic heterocyclic group (these groups may be unsubstituted or substituted) or is a hydrogen atom, a halogen atom, a nitro group, a cyano group, a substituted or unsubstituted alkyl group, a substituted or unsubstituted alkenyl group, a substituted or unsubstituted alkynyl group, a substituted or unsubstituted acyl group, or a group represented by the formula: -OR²¹, -N(R²²) R²³, -C(=O)OR²¹, -S(O)ₙR²⁴, -C(=O)R²⁵, -C(=O)N(R²²)R²³, -N(R²⁶)C(=O)R²⁵, -S(O)₂N(R²²)R²³, -N(R²⁶)S(O)ₙR²⁴ or -N(R²⁶)C(=O)OR²¹;
   M is a group represented by the formula: -C (=O) OR³¹, -S (O)ₙOR³¹, -C (=O)N(R³²)R³³, -S (O)ₙN (R³²) R³³ or -N (R³⁴) S (O)ₙR³⁵, a tetrazol-5-yl group, a 1,2,4-triazol-3-yl group, a 1,2,4-triazol-5-yl group, an imidazol-2-yl group or an imidazol-4-yl group;
   Q is taken together with the group represented by the formula: -C=C- to which Q is bonded, to represent a benzene ring or a 5- or 6-membered heteroaromatic ring (these rings may be unsubstituted or substituted);
   A is a saturated or unsaturated monocyclic hydrocarbon ring group, a saturated or unsaturated polycyclic hydrocarbon ring group, a saturated or unsaturated monocyclic heterocyclic group, or a saturated or unsaturated polycyclic heterocyclic group (these groups may be unsubstituted or substituted);
   R¹¹, R²¹, R²², R²³, R²⁴, R²⁵, R²⁶, R³¹, R³², R³³, R³⁴ and R³⁵, which may be the same or different, are independently as follows (when any of them is present as two or more substituents, these substituents are independently as follow): a hydrogen atom, a substituted or unsubstituted alkyl group, a substituted or unsubstituted alkenyl group, a substituted or unsubstituted cycloalkyl group or a substituted or unsubstituted aralkyl group, each of a combination of R²² and R²³ and a combination of R³² and R³³ being able to be taken together with the nitrogen atom to which the combination is bonded, to represent a saturated 3- to 8-membered cyclic amino group which may contain other heteroatoms in the ring (said cyclic amino group may be unsubstituted or substituted), provided that each of R²⁴ and R³⁵ is not a hydrogen atom when the number of oxygen atoms (n) on the sulfur atom bonded to R²⁴ or R³⁵, respectively, is 1 or 2; and
   n is 0, 1 or 2 (when n is present as two or more suffixes, these suffixes are independently 0, 1 or 2), a prodrug of said compound, or a pharmaceutically acceptable salt of said compound or prodrug.
[2] A compound, a prodrug thereof or a pharmaceutically acceptable salt of the compound or prodrug according to [1], wherein X is a sulfur atom.
[3] A compound, a prodrug thereof or a pharmaceutically acceptable salt of the compound or prodrug according to [1] or [2], wherein Y² is a group represented by the formula: -S(O)₂-, -C(=O)- or -CH₂-.
[4] A compound, a prodrug thereof or a pharmaceutically acceptable salt of the compound or prodrug according to [1], [2] or [3], wherein Q is taken together with the group represented by the formula: -C=C- to which Q is bonded, to represent an unsubstituted or substituted o-phenylene group.
[5] A compound, a prodrug thereof or a pharmaceutically acceptable salt of the compound or prodrug according to any one of [1] to [4], wherein M is a group represented by the formula: -C(=O)OR³¹.
[6] A compound, a prodrug thereof or a pharmaceutically acceptable salt of the compound or prodrug according to any one of [1] to [5], wherein Y¹ is a substituted or unsubstituted C₁₋₆alkylene group.
[7] A compound, a prodrug thereof or a pharmaceutically acceptable salt of the compound or prodrug according to any one of [1] to [6], wherein R² is hydroxyl group, a cyano group, a halogen atom or an unsubstituted C₁₋₆alkyl group.
[8] A compound, a prodrug thereof or a pharmaceutically acceptable salt of the compound or prodrug according to any one of [1] to [7], wherein A is a 1-naphthyl group or a 2-naphthyl group.
[9] A compound, a prodrug thereof or a pharmaceutically acceptable salt of the compound or prodrug according to any one of [1] to [8], wherein in one or both of R¹ and R², Z is a saturated or unsaturated monocyclic hydrocarbon ring group, a saturated or unsaturated polycyclic hydrocarbon ring group, a saturated or unsaturated monocyclic heterocyclic group, or a saturated or unsaturated polycyclic heterocyclic group, and each of these groups is substituted by a group represented by the formula: -Y⁴-Z' in which
   Y⁴ is a single bond or a substituted or unsubstituted alkylene group (the -CH₂- groups of the alkylene group may be replaced by one or more substituents which may be the same or different and are selected from groups represented by the formulas: -O-, -S(O)ₙ-, -N (R¹¹) - and -C (=O) -, substituted or unsubstituted benzene rings, and substituted or unsubstituted cycloalkane rings (the -CH₂- groups in the cycloalkane ring may be replaced by one or more substituents which may be the same or different and are selected from groups represented by the formulas: -O-, -S(O)ₙ-, -N(R¹¹)- and -C(=O)-), and any adjacent two carbon atoms of the alkylene group may form a double bond or a triple bond);
   Z' is a saturated or unsaturated monocyclic hydrocarbon ring group, a saturated or unsaturated polycyclic hydrocarbon ring group, a saturated or unsaturated monocyclic heterocyclic group, or a saturated or unsaturated polycyclic heterocyclic group (each of these groups may be either unsubstituted or substituted by one or more substituents which may be the same or different and are selected from halogen atoms, nitro group, cyano group, alkyl groups, aralkyl groups, alkoxy groups and alkylenedioxy groups); and
   R¹¹ and n are as defined above (when either of them is present as two or more substituents or suffixes, respectively, these substituents or suffixes are independently as defined above).
[10] A compound, a prodrug thereof or a pharmaceutically acceptable salt of the compound or prodrug according to any one of [1] to [9], wherein Y¹, Y², Y³ and Y⁴ are independently a group represented by the formula: - (CH₂)ₚ- (CH₂)_{q}-, - (CH₂)ₚ-O- (CH₂)_{q}-, - (CH₂)ₚ₋S(O)ₙ-(CH₂)_{q}-, - (CH₂)ₚ-N(R¹¹) - (CH₂)_{q}-, - (CH₂)ₚ-C (=O) N (R¹¹) - (CH₂)_{q}-, - (CH₂)ₚ-N (R¹¹) C (=O) - (CH₂)_{q}-, - (CH₂)ₚ-C (=O) O-(CH₂)_{q}- , -(CH₂)ₚ-OC (=O) (CH₂)_{q}-, -(CH₂)ₚ-SO₂N (R¹¹) - (CH₂)_{q}-, - (CH₂)ₚ-N (R¹¹) SO₂- (CH₂)_{q}- or -(CH₂)ₚ-R¹²-(CH₂)_{q}-, which may be substituted or unsubstituted and in which
   each of p and q is such an integer that p + q is 0 to 6, -(CH₂)p- may form a double bond or a triple bond between its adjacent carbon atoms in the case of p being 2 or more, and -(CH₂)_{q}- may form a double bond or a triple bond between its adjacent carbon atoms in the case of q being 2 or more; and
   R¹² is a substituted or unsubstituted benzene ring, or a substituted or unsubstituted cycloalkane ring.
[11] A pharmaceutical composition comprising a compound, a prodrug thereof or a pharmaceutically acceptable salt of the compound or prodrug according to any one of [1] to [10].
[12] A chymase inhibitor comprising a compound, a prodrug thereof or a pharmaceutically acceptable salt of the compound or prodrug according to any one of [1] to [10].
[13] A pharmaceutical composition for the treatment of hypertension, cardiac failure, ischemic peripheral circulatory disturbance, myocardial ischemia, venous malfunction, cardiac failure advance after myocardiac infarction, diabetic nephropathy, nephritis, arteriosclerosis, hyperaldosteronism, scleroderma, glomerulosclerosis, renal failure, central nervous system diseases, Alzheimer's disease, hypomnesia, depression, sensory functional disorders, anxiety, tension, unpleasant mental condition, glaucoma, ocular hypertension, restenosis after PTCA, asthma, rhinitis, COPD or allergic diseases, which comprises a compound, a prodrug thereof or a pharmaceutically acceptable salt of the compound or prodrug according to any one of [1] to [10].

The various groups in the present invention are explained below. The following explanation applies to the case where each group is a portion of another group, unless otherwise specified.

Each of the terms "saturated or unsaturated monocyclic hydrocarbon ring group", "saturated or unsaturated polycyclic hydrocarbon ring group", "saturated or unsaturated monocyclic heterocyclic group" and "saturated or unsaturated polycyclic heterocyclic group" means a group formed by the conversion of one of the hydrogen atoms of the corresponding saturated or unsaturated monocyclic hydrocarbon ring, saturated or unsaturated polycyclic hydrocarbon ring, saturated or unsaturated monocyclic heterocyclic ring, or saturated or unsaturated polycyclic heterocyclic ring, respectively, explained below to a bond.

As the saturated or unsaturated monocyclic hydrocarbon ring, there may be exemplified 3- to 8-membered hydrocarbon rings such as cyclopropane, cyclobutane, cyclopentane, cyclohexane, cycloheptane, cyclooctane, cyclopentene, cyclohexene, cycloheptene, cyclooctene, benzene, etc.

As the saturated or unsaturated polycyclic hydrocarbon ring, there may be exemplified polycyclic hydrocarbon rings of 16 or less carbon atoms, such as indene, naphthalene, azulene, fluorene, phenalene, phenanthrene, anthracene, acephenanthrylene, 1,2-dihydronaphthalene, 6,7-dihydro-5H-benzocycloheptene, benzocyclooctene, 1,2,3,4-tetrahydronaphthalene, decahydronaphthalene, octahydro-1H-indene, etc.; and crosslinked polycyclic hydrocarbon rings of 12 or less carbon atoms, such as adamantane, bicyclo[2,2,2]octane, bicyclo[3,3,3]undecane, bicyclo[2,2,2]oct-2-ene, bicyclo[3,3,3]undec-2-ene, etc.

As the saturated or unsaturated monocyclic heterocyclic ring, there may be exemplified 3- to 8-membered unsaturated monocyclic heterocyclic rings containing 1 to 4 nitrogen atoms, 3- to 8-membered saturated monocyclic heterocyclic rings containing 1 to 4 nitrogen atoms, 3- to 8-membered unsaturated monocyclic heterocyclic rings containing an oxygen atom, 3- to 8-membered unsaturated monocyclic heterocyclic rings containing one or two sulfur atoms, 3- to 8-membered unsaturated monocyclic heterocyclic rings containing 1 to 3 nitrogen atoms and one or two oxygen atoms, 3- to 8-membered saturated monocyclic heterocyclic rings containing 1 to 3 nitrogen atoms and one or two oxygen atoms, 3- to 8-membered unsaturated monocyclic heterocyclic rings containing 1 to 3 nitrogen atoms and one or two sulfur atoms, 3- to 8-membered saturated monocyclic heterocyclic rings containing 1 to 3 nitrogen atoms and one or two sulfur atoms, and 3- to 8-membered unsaturated monocyclic heterocyclic rings containing an oxygen atom and one or two sulfur atoms.

The 3- to 8-membered unsaturated monocyclic heterocyclic rings containing 1 to 4 nitrogen atoms include, for example, pyrrole, pyrroline, pyridine, dihydropyridine, imidazole, pyrazole, imidazoline, pyrazine, pyrimidine, pyridazine, pyrazole, triazole and tetrazole.

The 3- to 8-membered saturated monocyclic heterocyclic rings containing 1 to 4 nitrogen atoms include, for example, pyrrolidine, piperidine, imidazolidine, pyrazolidine and piperazine.

The 3- to 8-membered unsaturated monocyclic heterocyclic rings containing an oxygen atom include, for example, furan and pyran.

The 3- to 8-membered unsaturated monocyclic heterocyclic rings containing one or two sulfur atoms include, for example, thiophene, dihydrodithiin and dihydrodithion.

The 3- to 8-membered unsaturated monocyclic heterocyclic rings containing 1 to 3 nitrogen atoms and one or two oxygen atoms include, for example, oxazole, oxadiazole and isoxazole.

The 3- to 8-membered saturated monocyclic heterocyclic rings containing 1 to 3 nitrogen atoms and one or two oxygen atoms include, for example, morpholine and oxazolidine.

The 3- to 8-membered unsaturated monocyclic heterocyclic rings containing 1 to 3 nitrogen atoms and one or two sulfur atoms include, for example, thiazole, isothiazole and thiadiazole.

The 3- to 8-membered saturated monocyclic heterocyclic rings containing 1 to 3 nitrogen atoms and one or two sulfur atoms include, for example, thiazolidine.

The 3- to 8-membered unsaturated monocyclic heterocyclic rings containing an oxygen atom and one or two sulfur atoms include, for example, dihydrooxathiin.

As the saturated or unsaturated polycyclic heterocyclic ring, there may be exemplified saturated or unsaturated fused heterocyclic rings containing 1 to 4 nitrogen atoms, unsaturated fused heterocyclic rings containing 1 to 3 nitrogen atoms and one or two oxygen atoms, unsaturated fused heterocyclic rings containing 1 to 3 nitrogen atoms and one or two sulfur atoms, unsaturated fused heterocyclic rings containing one or two oxygen atoms, unsaturated fused heterocyclic rings containing an oxygen atom and one or two sulfur atoms, and unsaturated fused heterocyclic rings containing one or two sulfur atoms.

The saturated or unsaturated fused heterocyclic rings containing 1 to 4 nitrogen atoms include, for example, indole, isoindole, indoline, quinoline, isoquinoline, quinolizine, indazole, phthalazine, naphthyridine, quinoxaline, quinazoline, cinnoline, carbazole, purine, pteridine, phenazine, carbolinine, phenanthridine, acridine, indoline, isoindoline, 1,2-dihydroisoquinoline, benzimidazole, imidazopyridine, benzotriazole, tetrahydroimidazopyridine, benz[b]azepine, benz[cd]indole, cyclohepta[cd]indole, pyrrolo[3,2,1-ij]quinoline, cyclohexa[b]pyridine, cyclohepta[b]pyridine, pyrrolo[1,2,3-de]quinoxaline, pyrrolo[3,2,1-hi]indole, pyrrolo[3,2,1-jk][1]benzazepine, pyrrolo[3,2,1-kl][1]benzazocine, pyrrolo[3,2,1-kl]benzo[e][1,4]diazocine, 1,2,3,4-tetrahydroquinoline, 1,2,3,4-tetrahydroisoquinoline, decahydroquinoline, decahydroisoquinoline, octahydroindole, quinuclidine, 1-azabicyclo[2,2,1]heptane and 1-azabicyclo[3,2,1]octane.

The unsaturated fused heterocyclic rings containing 1 to 3 nitrogen atoms and one or two oxygen atoms include, for example, benzoxazole, benzoxadiazole, phenoxazine, pyrrolo[1,2,3-de][1,4]benzoxazine, pyrrolo[2,1-c][1,4]benzoxazine and pyrrolo[3,2,1-kl]benz[e][4,1]oxazocine. Preferable examples thereof are benzoxazole, pyrrolo[1,2,3-de][1,4]benzoxazine, pyrrolo[2,1-c][1,4]benzoxazine and pyrrolo[3,2,1-kl]benz[e][4,1]oxazocine.

The unsaturated fused heterocyclic rings containing 1 to 3 nitrogen atoms and one or two sulfur atoms include, for example, benzothiazole, benzothiadiazole, 1,4-benzothiazine and phenothiazine. Preferable examples thereof are benzothiazole and 1,4-benzothiazine.

The unsaturated fused heterocyclic rings containing one or two oxygen atoms include, for example, benzofuran, dihydrobenzofuran, chromene, isobenzofuran, xanthene, isochroman, chroman and benz[b]oxepine. Preferable examples thereof are benzofuran and benz[b]oxepine.

The unsaturated fused heterocyclic rings containing an oxygen atom and one or two sulfur atoms include, for example, 1,4-benzoxathiin and phenoxathiin.

The unsaturated fused heterocyclic rings containing one or two sulfur atoms include, for example, benzothiophene, benzothiin, benzothiopyran, thiochroman and thianthrene. Preferable examples thereof are benzothiophene, benzothiopyran and thiochroman.

As the 5- or 6-membered heteroaromatic ring which Q forms together with the -C=C- group to which Q is bonded, i.e., the partial structure represented by the formula: 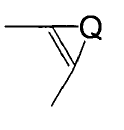 there may be exemplified groups formed by the conversion of hydrogen atoms on the adjacent carbon atoms of a 5- or 6-membered heteroaromatic ring containing one or two nitrogen atoms, zero or one oxygen atom and/or zero or one sulfur atom, to bonds. More specifically, there may be exemplified groups formed by the conversion of hydrogen atoms on the adjacent carbon atoms of pyridine, pyrazine, pyridazine, pyrimidine, pyrrole, imidazole, pyrazole, thiophene, thiazole, isothiazole, furan, oxazole or isoxazole, to bonds.

More specific examples of the benzene ring or the 5- or 6-membered heteroaromatic ring are those represented by the following formulas:

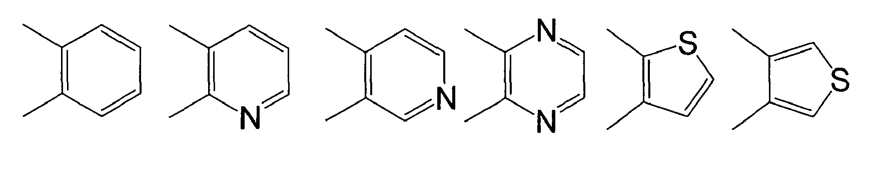

As the alkyl group, lower alkyl groups may be exemplified. The lower alkyl groups include, for example, linear or branched alkyl groups of 6 or less carbon atoms, such as methyl, ethyl, propyl, 2-propyl, butyl, 2-butyl, 2-methylpropyl, 1,1-dimethylethyl, pentyl, hexyl, heptyl, octyl, etc.

As the cycloalkyl group, there may be exemplified 3- to 8-membered cycloalkyl groups such as cyclopropyl, cyclobutyl, cyclopentyl, cyclohexyl, cycloheptyl, 2-methylcyclohexyl, etc.

As the alkenyl group, lower alkenyl groups may be exemplified. The lower alkenyl groups include, for example, linear or branched alkenyl groups of 6 or less carbon atoms, such as vinyl, allyl, propenyl, 2-propenyl, butenyl, pentenyl, hexenyl, etc.

As the alkynyl group, lower alkynyl groups may be exemplified. The lower alkynyl groups include, for example, linear or branched alkynyl groups of 6 or less carbon atoms, such as ethynyl, propargyl, butynyl, pentynyl, etc.

As the alkoxy group, there may be exemplified groups formed by bonding of an oxygen atom to the bond of each of the above-exemplified alkyl groups.

As the alkylenedioxy group, there may be exemplified groups formed by bonding of an oxygen atom to each of the two bonds of each of the above-mentioned alkylene groups.

The halogen atom includes, for example, iodine, fluorine, chlorine and bromine atoms.

As the acyl group, there may be exemplified formyl group; alkanoyl groups of 2 to 6 carbon atoms, such as acetyl, propanoyl, etc.; cycloalkanecarbonyl groups of 4 to 7 carbon atoms, such as cyclopropanecarbonyl, cyclobutanecarbonyl, cyclopentanecarbonyl, cyclohexanecarbonyl, etc.; cycloalkenecarbonyl groups of 3 to 6 carbon atoms, such as cyclopentenecarbonyl, cyclohexenecarbonyl, etc.; aroyl groups of 6 to 10 carbon atoms, such as benzoyl, toluoyl, naphthoyl, etc.; saturated heterocyclic ring-carbonyl groups having a 5- or 6-membered saturated heterocyclic ring containing one or two heteroatoms selected from nitrogen atom, oxygen atom and sulfur atom, such as 2-piperidinecarbonyl, 3-morpholinecarbonyl, etc.; and heteroaromatic acyl groups having a 5- or 6-membered heteroaromatic ring containing one or two heteroatoms selected from nitrogen atom, oxygen atom and sulfur atom, such as furoyl, thenoyl, nicotinoyl, isonicotinoyl, etc.

As the aralkyl group, alkyl groups substituted by a phenyl group or a polycyclic hydrocarbon ring group may be exemplified.

As the alkylene group, lower alkylene groups may be exemplified. The lower alkylene groups include, for example, linear or branched alkylene groups of 6 or less carbon atoms, such as methylene, ethylene, trimethylene, tetramethylene, pentamethylene, hexamethylene, ethylethylene, etc.

As the benzene ring as a substituent for the -CH₂- group of the alkylene group for each of Y¹, Y², Y³ and Y⁴, o-, m- or p-phenylene may be exemplified. As the cycloalkane ring as the substituent, there may be exemplified rings formed by the conversion of two hydrogen atoms of a 3- to 8-membered cycloalkane ring such as cyclopropane, cyclobutane, cyclopentane, cyclohexane, cycloheptane, cyclooctane or the like, to bonds.

As to the substituent(s) of each of the alkyl group, the alkenyl group, the alkynyl group, the alkanoyl group, the alkyl portion of the aralkyl group, and the alkylene group, they may be substituted by one or more substituents which may be the same or different. The substituent(s) includes halogen atoms, nitro group, cyano group, cycloalkyl groups, acyl groups, phenyl group, naphthyl group, saturated or unsaturated monocyclic hydrocarbon rings, saturated or unsaturated polycyclic hydrocarbon rings, saturated or unsaturated monocyclic heterocyclic rings, saturated or unsaturated polycyclic heterocyclic rings, oxo group, thioxo group, and groups represented by the formulas: -OR⁶¹, -N(R⁶²)R⁶³, -C(=O)OR⁶¹, -S(O)ₙR⁶⁴, -C (=O) -R⁶⁵, -C(=O)N(R⁶²) R⁶³, -N (R⁶⁶) C (=O) -R⁶⁵, -S(O)₂N(R⁶²) R⁶³, -N (R⁶⁶) S (O)ₙ-R⁶⁴ and -N (R⁶⁶) C (=O) -O-R⁶¹.

Here, n is as defined above. Each of R⁶¹, R⁶², R⁶³, R⁶⁴, R⁶⁵ and R⁶⁶, which may be the same or different, is a hydrogen atom, an alkyl group, a cycloalkyl group or an aralkyl group, but R⁶⁴ is not a hydrogen atom when the number of oxygen atoms (n) on the sulfur atom bonded to R⁶⁴ is 1 or 2. R⁶² and R⁶³ may bind to each other to form, together with the nitrogen atom to which they are bonded, a saturated 3- to 8-membered cyclic amino group that may contain other heteroatoms in the ring.

As to the substituent(s) of each of the saturated or unsaturated monocyclic hydrocarbon ring, the saturated or unsaturated polycyclic hydrocarbon ring, the saturated or unsaturated monocyclic heterocyclic ring, the saturated or unsaturated polycyclic heterocyclic ring, the phenyl group, the aroyl group, the saturated heterocyclic ring-carbonyl group, the heteroaromatic acyl group, the aryl portion of the substituted aralkyl group, and the benzene ring or 5- or 6-membered heteroaromatic ring, which Q forms together with the group represented by the formula: -C=C- to which Q is bonded, they may be substituted by one or more substituents which may be the same or different. The substituent(s) include, for example, substituted or unsubstituted alkyl groups, substituted or unsubstituted alkenyl groups, substituted or unsubstituted alkynyl groups, substituted or unsubstituted cycloalkyl groups, alkylenedioxy groups, carboxyl group, halogen atoms, nitro group, cyano group, saturated or unsaturated monocyclic hydrocarbon rings, saturated or unsaturated polycyclic hydrocarbon rings, saturated or unsaturated monocyclic heterocyclic rings, saturated or unsaturated polycyclic heterocyclic rings, heterocyclic groups, acyl groups, (these saturated or unsaturated monocyclic hydrocarbon rings, saturated or unsaturated polycyclic hydrocarbon rings, saturated or unsaturated monocyclic heterocyclic rings, saturated or unsaturated polycyclic heterocyclic rings and acyl groups may be substituted by an alkyl group, an alkoxy group, an alkylenedioxy group or a halogen atom), and groups represented by the formulas: -OR⁵¹, -N (R⁵²) R⁵³, -C (=O) OR⁵¹ , -S (O)ₙR⁵⁴, -C (=O) -R⁵⁵, -C (=O) N (R⁵²) R⁵³, -N (R⁵⁶) C (=O) -R⁵⁵, -S(O)₂N(R⁵² R⁵³, -N (R⁵⁶) S (O)ₙ-R⁵⁴ and -N (R⁵⁶) C (=O) -O-R⁵¹. Here, n is as defined above. Each of R⁵¹, R⁵² R⁵³ R⁵⁴, R⁵⁵ and R⁵⁶, which may be the same or different, is a hydrogen atom, an alkyl group, a cycloalkyl group or an aralkyl group, but R⁵⁴ is not a hydrogen atom when the number of oxygen atoms (n) on the sulfur atom bonded to R⁵⁴ is 1 or 2. R⁵² and R⁵³ may bind to each other to form, together with the nitrogen atom to which they are bonded, a saturated 3- to 8-membered cyclic amino group that may contain other heteroatoms in the ring.

As the heteroatoms of the saturated 3- to 8-membered cyclic amino group which may contain other heteroatoms in the ring and which R²² and R²³; R³² and R³³; R⁵² and R⁵³; or R⁶² and R⁶³ form by their mutual bonding together with the nitrogen atom to which they are bonded, oxygen atom, nitrogen atom and sulfur atom may be exemplified. Specific examples of such a saturated 3- to 8-membered cyclic amino group are 3- to 8-membered ring groups containing 1 to 3 nitrogen atoms, and 3- to 8-membered ring groups containing a nitrogen atom and an oxygen atom. More specific examples thereof are 1-pyrrolidinyl, 1-piperidinyl, 1-piperazinyl, morpholino, and 1-(4-methyl)piperazinyl.

As the substituent(s) of each of the saturated 3- to 8-membered cyclic amino group, the cycloalkyl group, the cycloalkane ring, the cycloalkanecarbonyl group and the cycloalkenecarbonyl group, there may be exemplified alkyl groups and the same groups as the above-exemplified substituent(s) of the substituted alkyl group.

The compounds of the present invention include those having an optical center of asymmetry. Therefore, the compound having an optical center of asymmetry may be obtained as a racemic modification, or it may be obtained as an optically active substance when an optically active starting material is used. If necessary, the racemic modification obtained may be physically or chemically resolved into optical antipodes by a well-known method. Preferably, diastereomers are formed from the racemic modification by a reaction using a reagent for optical resolution. The diastereomers may be resolved by a well-known method such as fractional crystallization.

As the "prodrug", there may be exemplified those which are easily hydrolyzed in a living body to regenerate the compound of formula (1). For example, when the compound of formula (1) has a carboxyl group, examples of the prodrug are compounds obtained by converting the carboxyl group to an alkoxycarbonyl group, an alkylthiocarbonyl group or an alkylaminocarbonyl group.

For example, when the compound of formula (1) has an amino group, examples of the prodrug are compounds obtained by converting the amino group to an alkanoylamino group by substitution by the alkanoyl group, compounds obtained by converting the amino group to an alkoxycarbonylamino group by substitution by the alkoxycarbonyl group, and compounds obtained by converting the amino group to an acyloxymethylamino group or hydroxylamine.

For example, when the compound of formula (1) has a hydroxyl group, examples of the prodrug are compounds obtained by converting the hydroxyl group to an acyloxyl group by substitution by the above-exemplified acyl group, and compounds obtained by converting the hydroxyl group to a phosphoric ester or an acyloxymethyloxy group.

For example, when the compound of formula (1) has a sulfo group, examples of the prodrug are compounds obtained by converting the sulfo group to a sulfonic ester by substitution by an alkyl group.

Examples of the alkyl portion of the group used for such conversion to the prodrug are the above-exemplified alkyl groups. The alkyl groups may be substituted by, for example, an alkoxy group of 1 to 6 carbon atoms. Preferable examples to the alkyl portion are as follows.
(a) For example, in the case of compounds obtained by converting the carboxyl group to an alkoxycarbonyl group, the alkoxycarbonyl group includes alkoxycarbonyl groups (lower alkoxycarbonyl groups of, for example, 1 to 6 carbon atoms) such as methoxycarbonyl, ethoxycarbonyl, etc.; and alkoxycarbonyl groups (lower alkoxycarbonyl groups of, for example, 1 to 6 carbon atoms) substituted by an alkoxy group, such as methoxymethoxycarbonyl, ethoxymethoxycarbonyl, 2-methoxyethoxycarbonyl, 2-methoxyethoxymethoxycarbonyl, pivaloyloxymethoxycarbonyl, etc.
(b) For example, in the case of compounds obtained by converting the sulfo group to an alkoxysulfonyl group, the alkoxysulfonyl group includes alkoxysulfonyl groups (lower alkoxysulfonyl groups of, for example, 1 to 6 carbon atoms) such as methoxysulfonyl, ethoxysulfonyl, etc.; and alkoxysulfonyl groups (lower alkoxysulfonyl groups of, for example, 1 to 6 carbon atoms) substituted by an alkoxy group, such as methoxymethoxysulfonyl, ethoxymethoxysulfonyl, 2-methoxyethoxysulfonyl, 2-methoxyethoxymethoxysulfonyl, pivaloyloxymethoxysulfonyl, etc.

If necessary, the compound of formula (1) or the prodrug thereof may be converted to a pharmaceutically acceptable salt. As such a salt, there may be exemplified salts with mineral acids such as hydrochloric acid, hydrobromic acid, sulfuric acid, phosphoric acid, etc.; salts with organic carboxylic acids such as formic acid, acetic acid, fumaric acid, maleic acid, oxalic acid, citric acid, malic acid, tartaric acid, aspartic acid, glutamic acid, etc.; salts with sulfonic acids such as methanesulfonic acid, benzenesulfonic acid, p-toluenesulfonic acid, hydroxybenzenesulfonic acid, dihydroxybenzenesulfonic acid, etc.; and alkali metal salts such as sodium salt, potassium salt, etc.; alkaline earth metal salts such as calcium salt, magnesium salt, etc.; ammonium salt; triethylamine salt, pyridine salt, picoline salt, ethanolamine salt, dicyclohexylamine salt and N,N'-dibenzylethylenediamine salt.

Each of the compounds of formula (1), the prodrugs thereof and the pharmaceutically acceptable salts of the compounds or prodrugs may be in the form of an anhydride, hydrate or solvate.

When used as a pharmaceutical composition, the compound of the present invention may be orally or parenterally administered. That is, the compound of the present invention may be orally administered in a usual dosage form such as powder, granules, tablets, capsules, syrup, suspension or the like, or it may be parenterally administered, for example, by injection of a solution, emulsion or suspension prepared from the compound. It may be administered rectally in the form of a suppository. The compound of the present invention may be formulated into the above-exemplified suitable dosage form by blending the compound with conventional acceptable adjuvants such as a carrier, excipient, binder, stabilizer and diluent. When the compound of the present invention is used in the form of an injection, the injection may contain acceptable additives such as a buffer, solubilizer and tonicity agent. Although the dose and the number of administrations are varied depending on, for example, a disease to be cured, symptoms, age, body weight and administration route, the compound of the present invention may be administered to an adult in a dose of usually 0.1 to 2,000 mg, preferably 1 to 200 mg per day in one portion or several portions (for example, 2 to 4 portions).

The compound of formula (1) may be synthesized from a well-known compound by a combination of well-known synthesis processes, and may be synthesized, for example, by any of the following processes.

### Synthesis Process (A)

In general, the compound of the present invention may be synthesized by the following process. 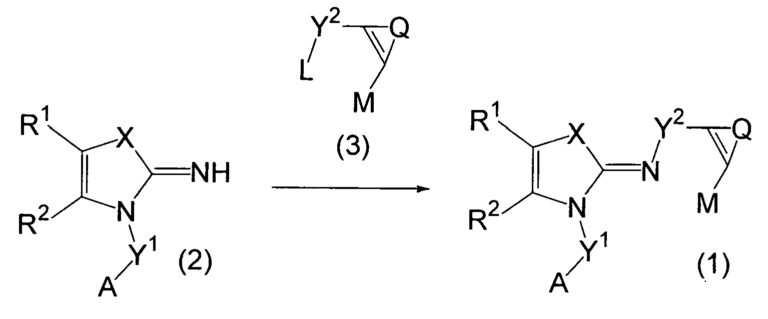 wherein X, R¹, R², Y¹, Y², A, Q and M are as defined above, and L is a halogen atom (e.g. chlorine atom or bromine atom), a substituted or unsubstituted alkylsulfonyloxy group (e.g. methanesulfonyloxy group) or a substituted or unsubstituted arylsulfonyloxy group (e.g. benzenesulfonyloxy group or p-toluenesulfonyloxy group).

The compound of formula (1) may be produced by reacting a compound of formula (2) with a compound of formula (3) in a solvent inert to the reaction (e.g. N,N-dimethylformamide, dimethyl sulfoxide, tetrahydrofuran or dichloromethane) in the presence of a base at 0°C to the boiling point of the solvent.

As the base used in the above reaction, the following may be exemplified. Also when the term "base" is hereinafter used without any other particular description, the following may be similarly exemplified as the base. That is, there may be exemplified inorganic bases (e.g. sodium hydrogencarbonate, potassium carbonate and sodium hydroxide), aqueous solutions thereof, organic bases (e.g. triethylamine and pyridine) and alkali metal hydrides (e.g. potassium hydride and sodium hydride). When any of these bases is used, there may be added a catalyst such as an iodide represented by n-tetrabutylammonium iodide, sodium iodide and potassium iodide, or 4-(N,N-dimethylamino)pyridine. In addition, when an alkaline aqueous solution is used, a phase transfer catalyst (e.g. n-tetrabutylammonium hydrogensulfate or n-tetrabutylammonium bromide) may be co-used.

### Synthesis Process (B)

A compound (1a) of formula (1) in which Y² represents the formula: -CO-Y^{2a}- (wherein Y^{2a} represents a portion of Y² which is other than the portion clearly shown as a specified group like -CO- in this case) may be produced, for example, by the following synthesis process: 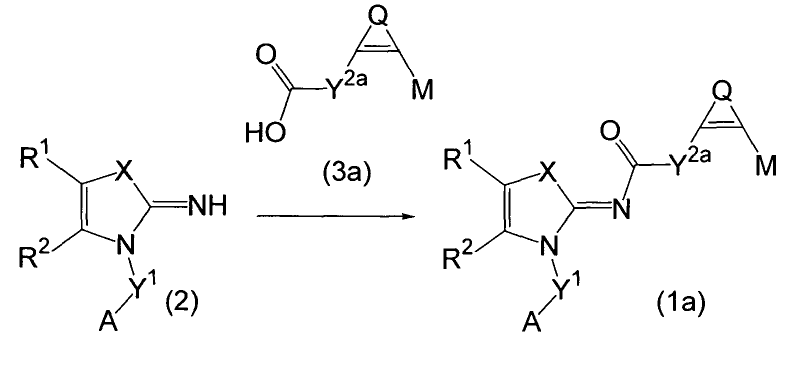 wherein X, R¹, R², Y¹, Y^{2a}, A, Q and M are as defined above.

The compound of formula (1a) may be produced by reacting a compound of formula (2) with a carboxylic acid of formula (3a) in an inert solvent in the presence of a condensing agent at room temperature or with heating. Alternatively, the production may be carried out also by reacting a compound of formula (2) with an acid halide or acid anhydride of a carboxylic acid of formula (3a) in an inert solvent in the presence of a base at 0°C to the boiling point of the solvent.

As the condensing agent used in the above reaction, the following may be exemplified. Also when the term "condensing agent" is hereinafter used without any other particular description, the following may be similarly exemplified as the condensing agent. That is, there may be exemplified dicyclohexylcarbodiimide (DCC), diisopropylcarbodiimide (DIPC), 1-ethyl-3-(3-dimethylaminopropyl)-carbodiimide (WSC), benzotriazol-1-yl-tris(dimethylamino)phosphonium hexafluorophosphide (BOP), diphenylphosphonyl diamide (DPPA) and N,N-carbonyldiimidazole (Angew. Chem. Int. Ed. Engl., 1962, 351). If necessary, there may be added additives such as N-hydroxysuccinimide (HOSu), 1-hydroxybenzotriazole (HOBt), 3-hydroxy-4-oxo-3,4-dihydro-1,2,3-benzotriazine (HOOBt), etc.

The solvent includes, for example, aromatic hydrocarbon solvents such as benzene, toluene, xylene, etc.; ether solvents such as tetrahydrofuran, 1,4-dioxane, etc.; halogenated hydrocarbon solvents such as dichloromethane, chloroform, 1,2-dichloroethane, etc.; amide solvents such as dimethylformamide, dimethylacetamide, etc.; basic solvents such as pyridine, etc.; and mixed solvents thereof. The base includes, for example, inorganic bases such as sodium hydroxide, potassium hydroxide, sodium carbonate, potassium carbonate, sodium hydrogencarbonate, etc.; and organic bases such as triethylamine, pyridine, etc. The acid halide includes acid chloride and acid bromide. As the acid anhydride, a mixed acid anhydride obtained by reaction with an alkyl chloroformate or the like may also be used.

### Synthesis Process (C)

A compound (1b) of formula (1) in which Y² represents the formula: -CO-NH-Y^{2a}- (wherein Y^{2a} represents a portion of Y² which is other than the portion clearly shown as a specified group like -CO-NH-in this case) may be produced, for example, by the following synthesis process: 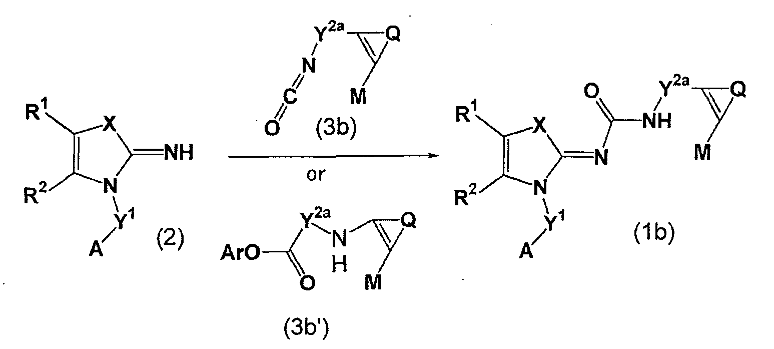 wherein X, R¹, R², Y¹, Y^{2a}, A, Q and M are as defined above, and Ar is a phenyl group or a nitrophenyl group such as p-nitrophenyl.

The compound of formula (1b) may be produced by reacting a compound of formula (2) with a compound of formula (3b) or (3b') in a solvent inert to the reaction (e.g. N,N-dimethylformamide, dimethyl sulfoxide, tetrahydrofuran or dichloromethane) in the presence or absence of a base at 0°C to the boiling point of the solvent.

### Synthesis Process (D)

A compound (1c) of formula (1) in which Y² represents the formula: -SO₂-NH-Y^{2a}- (wherein Y^{2a} represents a portion of Y² which is other than the portion clearly shown as a specified group like -SO₂-NH-in this case) may be produced, for example, by the following synthesis process (D1) or (D2):

### Synthesis process (D1)

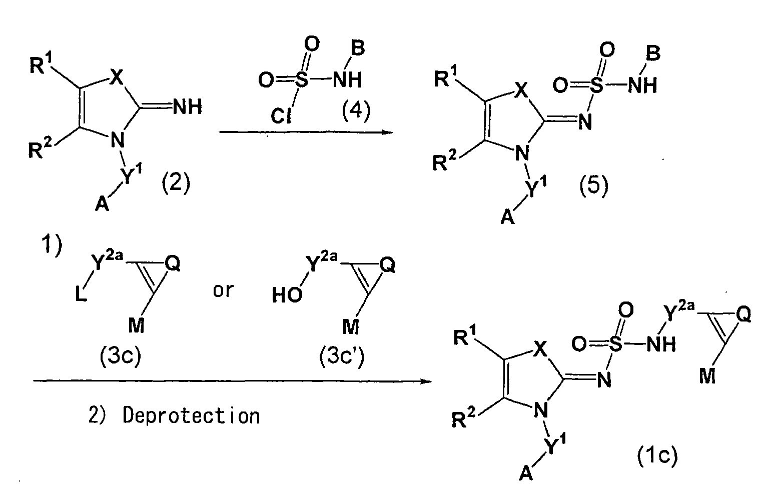

wherein X, R¹, R², Y¹, Y^{2a}, A, Q, L and M are as defined above, and B is a protective group such as tert-butoxycarbonyl.

The compound of formula (1c) may be produced from a compound of formula (2) according to the process described in literature (for example, Bioorg. Med. Chem., 1999, 9, 3103., Tetrahedron, 1993, 1, 65. or Nucleosides Nucleotides, 1995, 14, 8.). That is, a compound of formula (5) may be produced, for example, by reacting a compound of formula (2) with a compound of formula (4) in the presence or absence of a base at 0°C to room temperature in a solvent inert to the reaction (e.g. N,N-dimethylformamide, dimethyl sulfoxide, tetrahydrofuran or dichloromethane). Then, the compound of formula (1c) may be produced by reacting the compound of formula (5) obtained with a compound of formula (3c) in a solvent inert to this reaction in the presence a base at 0°C to the boiling point of the solvent. Alternatively, the compound of formula (1c) may be produced by reacting the compound of formula (5) with an alcohol of formula (3c') in an inert solvent at room temperature or with heating in the presence of, for example, diethyl azodicarboxylate and triphenylphosphine.

### Synthesis process (D2)

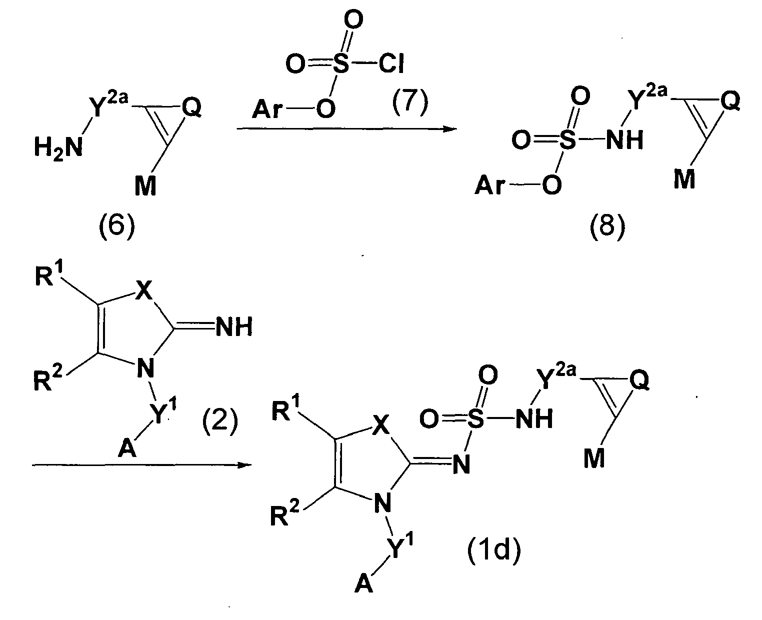

wherein X, R¹, R², Y¹, Y^{2a}, A, Q and M are as defined above, and Ar is a phenyl group or a nitrophenyl group such as p-nitrophenyl.

A compound of formula (1d) may be produced from an amine derivative of formula (6) according to the process described in literature (for example, J. Chem. Soc., Perkin Trans. 1, 2002, 4, 485.). That is, a compound of formula (8) may be produced, for example, by reacting the amine derivative of formula (6) with a compound of formula (7) in the presence or absence of a base at -78°C to room temperature in a solvent inert to the reaction (e.g. N,N-dimethylformamide, dimethyl sulfoxide, tetrahydrofuran or dichloromethane). Then, the compound of formula (1d) may be produced by reacting the compound of formula (8) obtained with a compound of formula (2) in a solvent inert to this reaction in the presence a base at 0°C to the boiling point of the solvent.

### Synthesis Process (E)

The compound of formula (2) used in the above synthesis processes (A) to (D) may be synthesized from a well-known compound by a combination of well-known synthesis processes and may be synthesized by the following process: 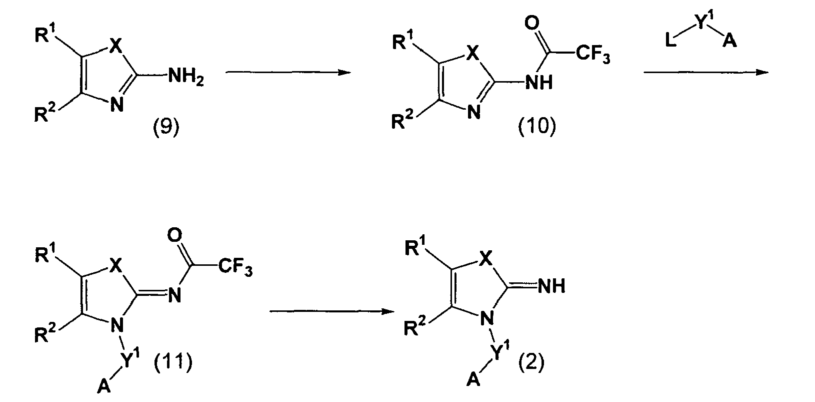 wherein X, R¹, R², Y¹, A and L are as defined above.

The compound of formula (2) may be produced from an azole derivative of formula (9) according to the process described in literature (for example, Bioorg. Med. Chem., 1996, 6, 1469.). That is, a compound of formula (10) may be produced, for example, by reacting the azole derivative of formula (9) with trifluoroacetic anhydride in the presence or absence of a base at 0°C to room temperature in a solvent inert to the reaction (e.g. toluene, tetrahydrofuran, dichloromethane or N,N-dimethylformamide). Subsequently, a compound of formula (11) may be produced by reacting the compound of formula (10) obtained with a compound of the formula: L-Y¹-A in a solvent inert to this reaction in the presence of a base at 0°C to the boiling point of the solvent. Thereafter, the compound of formula (2) may be produced, for example, by subjecting the compound of formula (11) to alkali hydrolysis using a hydroxide (e.g. sodium hydroxide or potassium hydroxide), in an alcohol solvent (e.g. methanol or ethanol).

In addition, the compound of formula (1) may be produced from the above-mentioned compound of formula (9) without producing the above-mentioned compound of formula (2) as an intermediate. As such a process, the following synthesis processes (F) and (G) may be exemplified.

### Synthesis Process (F)

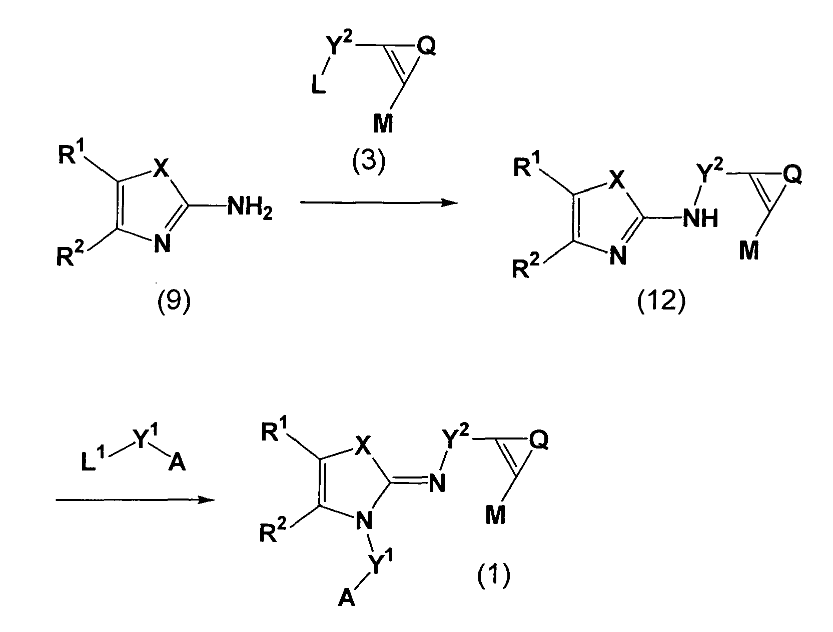

wherein X, R¹, R², Y¹, Y², A, Q, L and M are as defined above, and L¹ has the same meaning as that of L defined above.

A compound of formula (12) may be produced by reacting an azole derivative of formula (9) with a compound of formula (3) in a solvent inert to the reaction (e.g. N,N-dimethylformamide, dimethyl sulfoxide, tetrahydrofuran or dichloromethane) in the presence of a base at 0°C to the boiling point of the solvent. Then, the compound of formula (1) may be produced by reacting the compound of formula (12) obtained with a compound of the formula: L¹-Y¹-A in a solvent inert to this reaction in the presence of a base at 0°C to the boiling point of the solvent.

### Synthesis Process (G)

A compound (1a) of formula (1) in which Y² represents the formula: -CO-Y^{2a}- (wherein Y^{2a} represents a portion of Y² which is other than the portion clearly shown as a specified group like -CO- in this case) may be produced also by, for example, the following synthesis process: 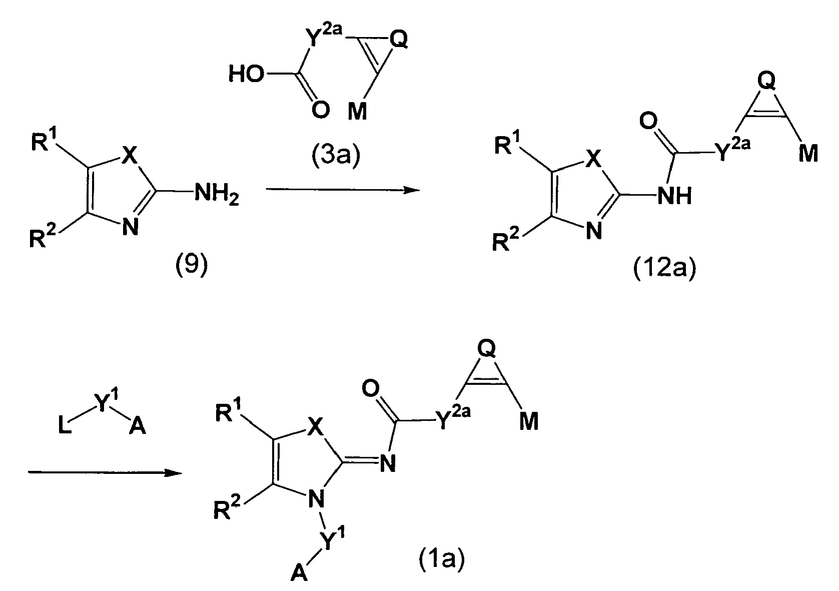 wherein X, R¹, R², Y¹, Y^{2a}, A, Q, L and M are as defined above.

A compound of formula (12a) may be produced by reacting an azole derivative of formula (9) with a carboxylic acid of formula (3a) in an inert solvent in the presence of a condensing agent at room temperature or with heating. Alternatively, the production may be carried out by reacting an azole derivative of formula (9) with an acid halide or acid anhydride of a carboxylic acid of formula (3a) in an inert solvent in the presence of a base at 0°C to the boiling point of the solvent. Then, the compound of formula (1a) may be produced by reacting the compound of formula (12a) obtained with a compound of the formula: L-Y¹-A in a solvent inert to this reaction in the presence of a base at 0°C to the boiling point of the solvent.

The starting compound used in the above synthesis process may be produced by reacting compounds having a side chain bonded thereto, to form a thiazole ring or an oxazole ring. It may be produced, for example, by the following process.

### Synthesis Process (H)

A thiazole derivative (9A), i.e., an azole derivative of formula (9) in which X represents the formula: -S-, may be synthesized from a well-known compound by a combination of well-known synthesis processes and may be synthesized, for example, by the following process: 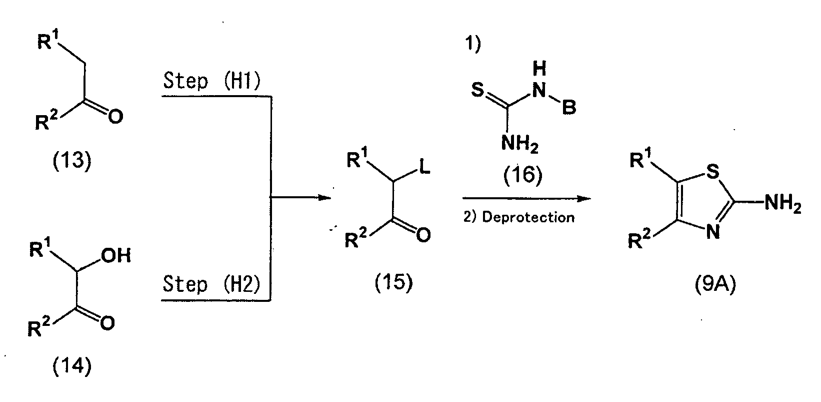 wherein R¹, R² and L are as defined above, and B is a hydrogen atom or a protective group such as tert-butoxycarbonyl or triphenylmethyl.

A compound of formula (15) may be produced through the following step (H1) or (H2).

### Step (H1) :

The compound of formula (15) may be synthesized from a compound of formula (13) according to the process described in literature (for example, J. Med. Chem., 1987, 30, 494. or Tetrahedron Lett., 2000, 41, 9741.). That is, the compound of formula (15) may be produced by reacting the compound of formula (13) with a halogenating agent such as bromine or iodine in a solvent inert to the reaction (e.g. acetic acid, chloroform, tetrahydrofuran, toluene or acetonitrile) at 0°C to the boiling point of the solvent.

### Step (H2):

The compound of formula (15) may be synthesized by reacting a compound of formula (14) with a sulfonylating agent such as methanesulfonyl chloride in a solvent inert to the reaction (e.g. methylene chloride, tetrahydrofuran, toluene or acetonitrile) in the presence or absence of a base at 0°C to the boiling point of the solvent.

The compound of formula (9A) may be synthesized from the compound of formula (15) according to the process described in literature (for example, J. Med. Chem., 1987, 30, 494. or Synth. Commun., 2002, 32, 1671.). That is, the thiazole derivative of formula (9A) may be produced by reacting the compound of formula (15) with a thiourea derivative of formula (16) in a solvent inert to the reaction (e.g. acetic acid, chloroform, 1,4-dioxane or tetrahydrofuran) at 0°C to the boiling point of the solvent, and if necessary, removing B.

The halogenating agent used in step (H1) includes, for example, bromine, iodine, pyridinium bromide perbromide and 5,5-dibromobarbituric acid.

### Synthesis Process (J)

As the compounds of formulas (2), (12) and (1) as a starting material, an intermediate for synthesis and the compound of the present invention, respectively, in the synthesis processes described above, each of compounds (2A), (12A) and (1A) in which X represents the formula: -S- may be synthesized from a well-known compound by a combination of well-known synthesis processes and may be synthesized, for example, by the following process (J1), (J2) or (J3), respectively.

### Synthesis process (J1)

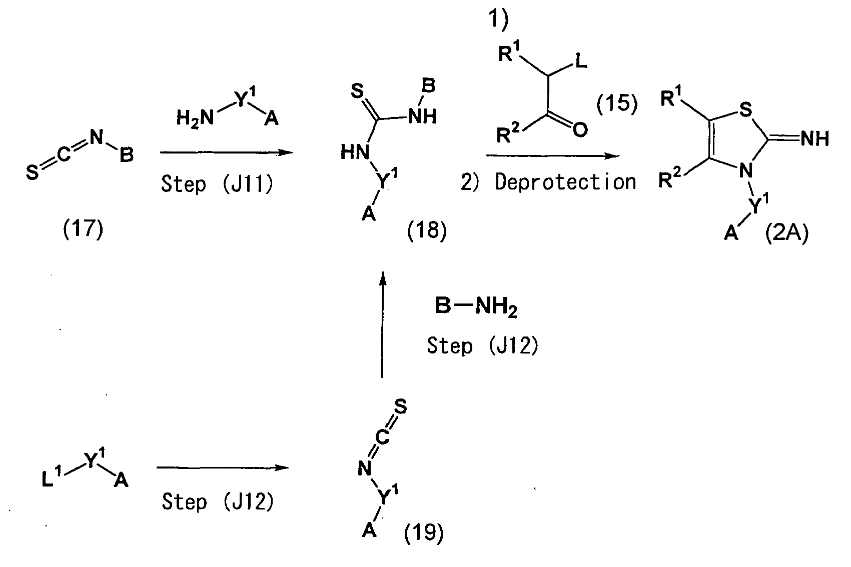

wherein R¹, R², Y¹, A, L and B are as defined above, and L¹ has the same meaning as that of L defined above.

A compound of formula (18) may be produced through the following step (J11) or (J12).

### Step (J11) :

The thiourea derivative of formula (18) may be produced by reacting an isothiocyanate derivative of formula (17) with a compound of the formula: NH₂-Y¹-A in a solvent inert to the reaction (e.g. N,N-dimethylformamide, acetonitrile, tetrahydrofuran or dichloromethane) in the presence or absence of a base at 0°C to the boiling point of the solvent.

### Step (J12):

An isothiocyanate derivative of formula (19) may be produced by reacting a compound of the formula: L¹-Y¹-A with a thiocyanate (e.g. potassium thiocyanate, sodium thiocyanate or ammonium thiocyanate) in a solvent inert to the reaction (e.g. acetonitrile, tetrahydrofuran, dichloromethane or acetic acid) in the presence or absence of a base at 0°C to the boiling point of the solvent. Then, the thiourea derivative of formula (18) may be produced by reacting the isothiocyanate derivative of formula (19) obtained with a compound of the formula: B-NH₂ in a solvent inert to this reaction (e.g. acetonitrile, tetrahydrofuran or dichloromethane) in the presence or absence of a base at 0°C to the boiling point of the solvent.

Subsequently, the compound of formula (2A) may be produced by reacting the compound of formula (18) obtained with a compound of formula (15) under the same conditions as in the above-mentioned synthesis process of the compound of formula (9A) according to the method described in literature (for example, J. Med. Chem., 1987, 30, 494. or Synth. Commun., 2002, 32, 1671.), and then carrying out deprotecting reaction when the group represented by B in the formula is a protective group.

### Synthesis process (J2)

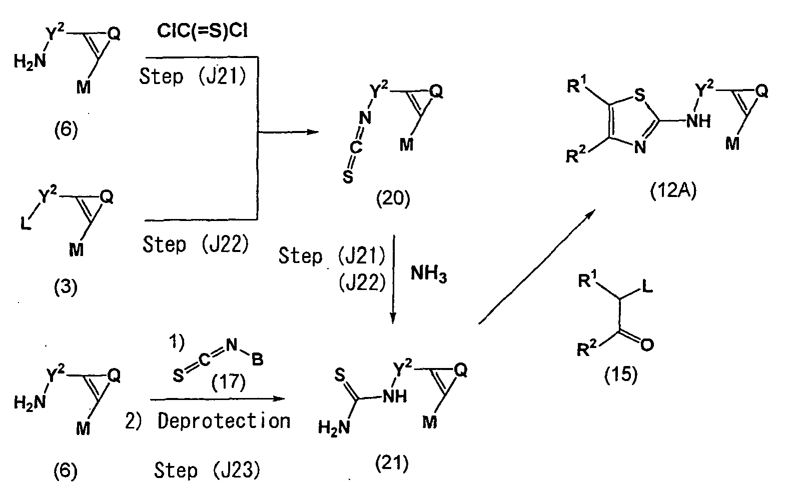

wherein R¹, R², Y², L, Q, M and B are as defined above.

A compound of formula (21) may be produced through the following step (J21), (J22) or (J23).

### Step (J21):

An isothiocyanate derivative of formula (20) may be produced by reacting an amine derivative of formula (6) with thiophosgene in a solvent inert to the reaction (e.g. acetonitrile, tetrahydrofuran or dichloromethane) in the presence or absence of a base at 0°C to the boiling point of the solvent. Then, the thiourea derivative of formula (21) may be produced by reacting the compound of formula (20) obtained with ammonia in a solvent inert to this reaction (e.g. tetrahydrofuran, dichloromethane, chloroform, methanol or ethanol) in the presence or absence of a base at 0°C to the boiling point of the solvent.

### Step (J22):

An isothiocyanate derivative of formula (20) may be produced by reacting a compound of formula (3) with a thiocyanate (e.g. potassium thiocyanate, sodium thiocyanate or ammonium thiocyanate) in the same manner as in step (J12) in the above-mentioned synthesis process (J1). Then, the thiourea derivative of formula (21) may be produced by reacting the isothiocyanate derivative of formula (20) obtained with ammonia in the same manner as in the above step (J21).

### Step (J23):

The compound of formula (21) may be produced by reacting a compound of formula (6) with a compound of formula (17) in the same manner as in step (J11) in the above-mentioned synthesis process (J1), and then carrying out deprotecting reaction when the group represented by B in the formula is a protective group.

Subsequently, the compound of formula (12A) may be produced by reacting the compound of formula (21) obtained with a compound of formula (15) in the same manner as in the above-mentioned synthesis process (J1).

### Synthesis process (J3)

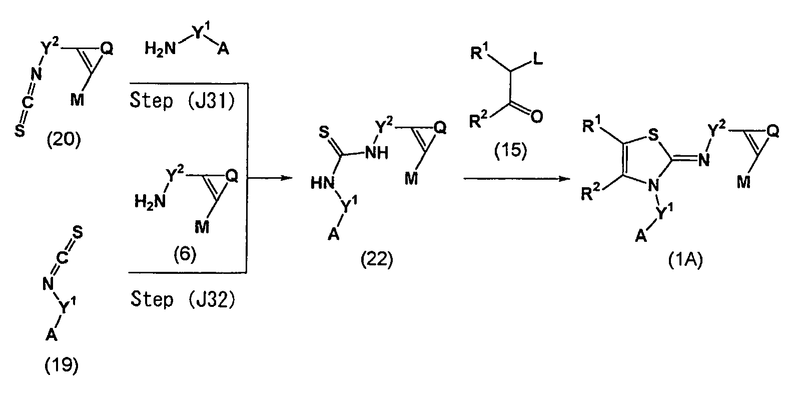

wherein R¹, R², Y¹, Y², L, Q, A and M are as defined above.

A compound of formula (22) may be produced through the following step (J31) or (J32).

### Step (J31):

The thiourea derivative of formula (22) may be produced by reacting a compound of formula (20) with a compound of the formula: NH₂-Y¹-A in the same manner as in step (J12) in the above-mentioned synthesis process (J1).

### Step (J32):

The thiourea derivative of formula (22) may be produced by reacting a compound of formula (19) with a compound of formula (7) in the same manner as in step (J21) in the above-mentioned synthesis process (J2), or step (J12) in the above-mentioned synthesis process (J1) .

Then, the compound of formula (1A) may be produced by reacting the compound of formula (22) obtained with a compound of formula (15) in the same manner as in the above-mentioned synthesis process (J1) or (J2).

### Synthesis Process (K)

As the azole derivative of formula (9) used as a starting compound in the above-mentioned synthesis processes (E) to (G), an oxazole derivative (9B) in which X represents the formula: -O- may be synthesized from a well-known compound by a combination of well-known synthesis processes. It may be synthesized, for example, by the following process (K1) or (K2).

### Synthesis process (K1)

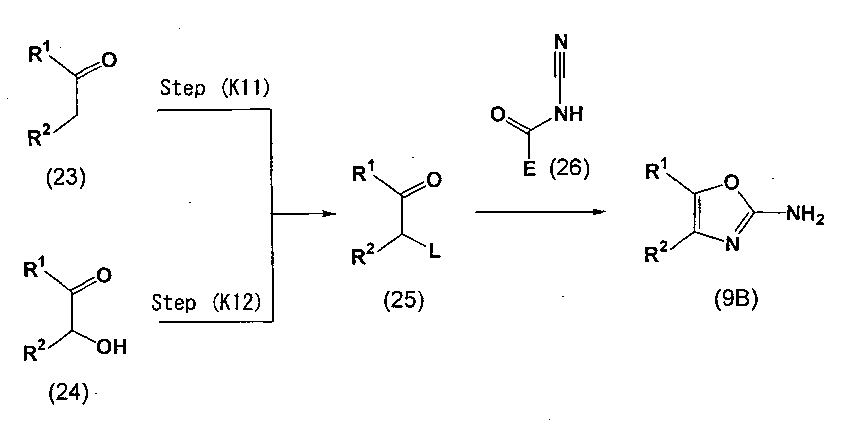

wherein R¹, R² and L are as defined above, and E is a -NH₂ group, a -NHPh group or an alkoxy group such as ethoxy group.

A compound of formula (25) may be produced through the following step (K11) or (K12).

### Step (K11) :

The compound of formula (25) may be synthesized from a compound of formula (23) according to the synthesis process of the above-mentioned compound of formula (9A) (step (H1) in synthesis process (H)). That is, the compound of formula (25) may be produced by reacting the compound of formula (23) with a halogenating agent such as bromine or iodine in a solvent inert to the reaction at 0°C to the boiling point of the solvent.

### Step (K12):

The compound of formula (25) may be synthesized from a compound of formula (24) according to the synthesis process of the above-mentioned compound of formula (9A) (step (H2) in synthesis process (H)). That is, the compound of formula (25) may be produced by reacting the compound of formula (24) with a sulfonylating agent such as methanesulfonyl chloride in a solvent inert to the reaction in the presence or absence of a base at 0°C to the boiling point of the solvent.

Then, the 2-aminooxazole derivative of formula (9B) may be synthesized by the use of the compound of formula (25) according to the process described in literature (for example, Chem. Ber., 1966, 99, 2110.). That is, the compound of formula (9B) may be produced by reacting the compound of formula (25) with a compound of formula (26) in a mixed solvent of an alcohol solvent (e.g. methanol, ethanol or 2-propanol) and water in the presence of sodium acetate at room temperature to the boiling point of the solvent.

### Synthesis process (K2)

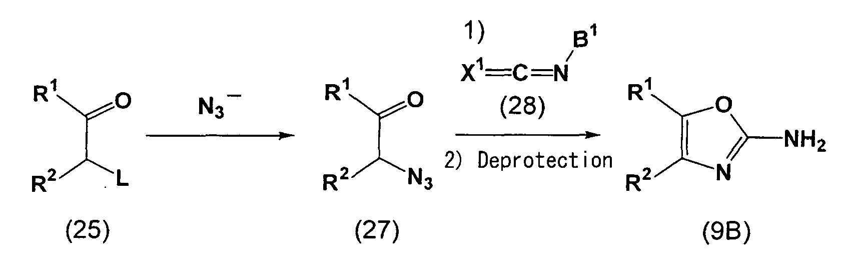

wherein R¹, R² and L are as defined above, X¹ is an oxygen atom or a sulfur atom, and B¹ is a protective group such as triphenylmethyl.

The compound of formula (9B) may be synthesized via a compound of formula (27) according to the process described in literature (for example, Synthesis, 1993, 54., Tetrahedron, 1999, 55, 14701., or Org. Lett., 2002, 4, 54.). That is, the carbonyl α-azide derivative of formula (27) may be produced by reacting a compound of formula (25) with an azide (e.g. sodium azide) in a solvent inert to the reaction (e.g. N,N-dimethylformamide, acetone, acetonitrile or tetrahydrofuran) at 0°C to the boiling point of the solvent. Subsequently, the compound of formula (9B) may be produced by reacting the compound of formula (27) obtained with an isocyanate or isothiocyanate derivative of formula (28) in a solvent inert to this reaction (e.g. dichloromethane, diethyl ether, tetrahydrofuran or 1,4-dioxane) in the presence of triphenylphosphine or tributylphosphine at 0°C to the boiling point of the solvent, and then removing the protective group B¹.

### Synthesis Process (L2)

As the compound of formula (12) obtained as an intermediate for synthesis in the above-mentioned synthesis process, an oxazole derivative (12B) in which X represents the formula: -O- may be synthesized from a well-known compound by a combination of well-known synthesis processes and may be synthesized, for example, by the following process. 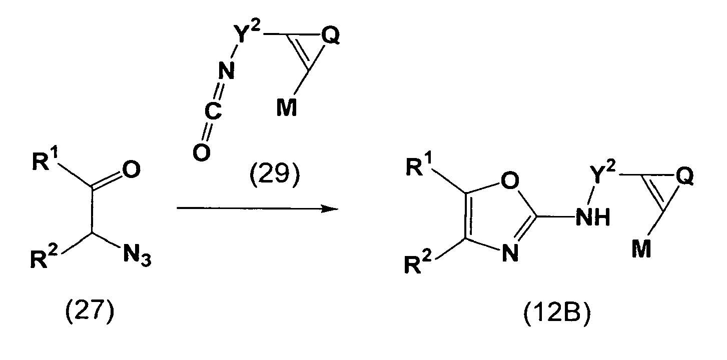 wherein R¹, R², Y², Q and M are as defined above.

The oxazole derivative of formula (12B) may be produced by reacting a compound of formula (27) with a compound of formula (29) in the same manner as in the above-mentioned synthesis process (K2).

In the reactions explained above, not only when a specified protective group is exemplified, but also when each starting compound has a reactive group such as carboxyl group, hydroxyl group or amino group, the reactive group is previously protected with a suitable protective group and the protective group is removed after carrying out the reaction, whereby a desired compound may be produced. As to methods for the protection and the deprotection, they may be carried out by the methods described in literature (for example, Green, T.W. and Wuts, P.G.M., Protective Groups in Organic Synthesis, John Wiley & Sons, Inc. (1999)), depending on each protective group.

Specifically, when the substituent represented by M of any of the compounds of formulas (1), (1a), (1b), (1c) and (1d) is protected with a protective group, the protective group is removed, for example, by a method using an aqueous alkali solution of sodium hydroxide or the like, or an acid such as hydrochloric acid or trifluoroacetic acid.

The protective groups represented by B and B¹ in the formulas shown above are removed, for example, by a method using an acid such as hydrochloric acid, formic acid or trifluoroacetic acid.

### EXAMPLES

The present invention is more concretely illustrated below with reference examples, working examples and a test example, which should not be construed as limiting the scope of the invention. The nomenclature of compounds shown in the reference examples and working examples mentioned below is not always based on IUPAC.

LC/MS conditions are as follows and analysis was carried out by adopting such a method in all cases. Column: octadecyl-chemically-bonded type silica (ODS)
(CombiScreen ODS-A, a trade name, (YMC Co., Ltd.))
50 mm (length) x 4.6 mm (inside diameter), particle size 5 µm, pore 120 angstrom
Flow rate: 3.5 ml/min
Mobile phase:
mobile phase A (a 0.05% aqueous trifluoroacetic acid solution)
mobile phase B (a 0.035% solution of trifluoroacetic acid in acetonitrile)

The ratio between mobile phases A and B during each period is as follows:

| Elapsed time (min) : | Mobile phase A / mobile phase B |
|---|---|
| 0 → 0.5 : | 90/10 |
| 0.5 → 4.2 : | 90/10 → 1/99 |
| 4.2 → 4.4 : | 1/99 |
| 4.4 → 4.8 : | 1/99 → 99/1 |
| 4.8 → 6.3 : | 99/1 |
| 6.3 → 6.4 : | 99/1 → 100/0 |

### Reference Example 1

### Synthesis of 2,2,2-trifluoro-N-(5-methyl-1,3-thiazol-2-yl)acetamide

Trifluoroacetic anhydride (6.18 ml, 43.8 mmol) was added dropwise to a solution (150 ml) of 2-amino-5-methylthiazole (5.00 g, 43.8 mmol) in toluene at 0°C and stirred for 2 hours. After completion of the reaction, a saturated aqueous sodium chloride solution was added thereto, followed by extraction with chloroform. The organic layer was washed successively with a 2M aqueous hydrochloric acid solution, a saturated aqueous sodium chloride solution, a saturated aqueous sodium hydrogencarbonate solution and a saturated aqueous sodium chloride solution and then dried over anhydrous sodium sulfate. The solvent was distilled off under reduced pressure and the thus obtained solid was washed with a hexane-diethyl ether mixed solvent to obtain the title compound (5.90 g, 69.1%).; ¹H-NMR(DMSO-d₆) 13.76 (brs, 1H), 7.35 (br, 1H), 2.31 (br,3H). LC/MS (M+1, retention time): 211.0, 3.07 min.

### Reference Example 2

### Synthesis of 2,2,2-trifluoro-N-[(2Z)-5-methyl-3-(1-naphthylmethyl)-1,3-thiazol-2(3H)-ylidene]acetamide

Potassium carbonate (4.24 g, 30.8 mmol) and a catalytic amount of potassium iodide were added to a solution of 2,2,2-trifluoro-N-(5-methyl-1,3-thiazol-2-yl)acetamide (3.00 g, 15.4 mmol) and 1-(chloromethyl)-naphthalene (2.86 g, 16.15 mmol) in N,N-dimethylformamide (15 ml) and the resulting mixture was stirred at room temperature for 1.5 hours. After completion of the reaction, a saturated aqueous sodium chloride solution was added thereto, followed by extraction with ethyl acetate. The organic layer was washed with water and a saturated aqueous sodium chloride solution and then dried over anhydrous sodium sulfate. The solvent was distilled off under reduced pressure and the thus obtained solid was washed with a hexane-diethyl ether mixed solvent to obtain the title compound (4.27 g, 79.2%).; ¹H-NMR(DMSO-d₆) 8.20-8.30 (m,1H), 7.90-8.05 (m,2H), 7.45-7.60 (m,3H), 7.30-7.45 (m,2H), 5.88 (s,2H), 2.28 (s,3H). LC/MS (M+1, retention time): 439.0, 4.13 min.

### Reference Example 3

### Synthesis of 5-methyl-3-(1-naphthylmethyl)-1,3-thiazol-2(3H)-imine

In a mixture of tetrahydrofuran (10 ml) and methanol (10 ml) was dissolved 2,2,2-trifluoro-N-[(2Z)-5-methyl-3-(1-naphthylmethyl)-1,3-thiazol-2(3H)-ylidene]acetamide (2.76 g, 7.89 mmol), followed by adding thereto a 2M aqueous sodium hydroxide solution (10 ml) at room temperature, and the resulting mixture was refluxed for 1 hour. After completion of the reaction, a saturated aqueous sodium chloride solution was added thereto, followed by extraction with chloroform. The organic layer was washed with a saturated aqueous sodium chloride solution and then dried over anhydrous sodium sulfate. The solvent was distilled off under reduced pressure to obtain the title compound (1.75 g, 87.3%).; ¹H-NMR(CDCl₃) 8.04-8.10 (m,1H), 7.80-7.92 (m,2H), 7.37-7.60 (m,4H), 5.84-5.87 (m,1H) , 5.26 (s,2H), 1.92 (d,3H,J=1.5Hz). LC/MS (M+1, retention time): 255.2, 3.26 min.

### Example 1

(a) Synthesis of methyl 2-({[(2Z)-5-methyl-3-(1-naphthylmethyl)-1,3-thiazol-2(3H)-ylidene]amino}-sulfonyl)benzoate
   Methyl 2-(chlorosulfonyl)benzoate (203 mg, 0.865 mmol), diisopropylethylamine (0.27 ml, 1.57 mmol) and a catalytic amount of 4-dimethylaminopyridine were added to a solution (6 ml) of 5-methyl-3-(1-naphthylmethyl)-1,3-thiazol-2(3H)-imine (200 mg, 0.787 mmol) in dichloromethane at 0°C and stirred at room temperature for 1 hour. After completion of the reaction, the reaction solution was cooled to 0°C and a saturated aqueous sodium chloride solution was added thereto, followed by extraction with ethyl acetate. The organic layer was washed successively with a 2M aqueous hydrochloric acid solution, a saturated aqueous sodium chloride solution, a saturated aqueous sodium hydrogencarbonate solution and a saturated aqueous sodium chloride solution and then dried over anhydrous sodium sulfate. The solvent was distilled off under reduced pressure and the resulting residue was purified by a silica gel column chromatography (eluent: hexane/ethyl acetate = 7/3) to obtain the title compound (218 mg, 61.2%).; ¹H-NMR(CDCl₃) 8 . 10-8 . 18 (m,1H), 7.80-7.92 (m,3H), 7.30-7.62 (m,6H), 7.26-7.34 (m,1H), 6.24 (br,1H), 5.50 (s,2H), 3.91 (s,3H), 2.06 (br,3H). LC/MS (M+1, retention time): 453.0, 4.39 min.
(b) Synthesis of 2-({[(2Z)-5-methyl-3-(1-naphthylmethyl)-1,3-thiazol-2(3H)-ylidene]amino}sulfonyl)benzoic acid
   Methyl 2-({[(2Z)-5-methyl-3-(1-naphthylmethyl)-1,3-thiazol-2(3H)-ylidene]amino}sulfonyl)benzoate (180 mg, 0.331 mmol) was dissolved in a mixture of tetrahydrofuran (5 ml) and methanol (5 ml), followed by adding thereto a 2M aqueous sodium hydroxide solution (2 ml) at room temperature, and the resulting mixture was refluxed for 1 hour. After completion of the reaction, the reaction solution was cooled to 0°C, adjusted to a pH of about 5 with a 2M aqueous hydrochloric acid solution and a saturated aqueous sodium hydrogencarbonate solution, and then extracted with ethyl acetate. The organic layer was washed with a saturated aqueous sodium chloride solution and then dried over anhydrous sodium sulfate. The solvent was distilled off under reduced pressure to obtain the title compound (141 mg, 80.8%).; ¹H-NMR(DMSO-d₆) 7.70-8.00 (m,4H), 7.40-7.70 (m, 5H), 7.20-7.35 (m,2H), 6.96 (d,1H,J=1.5Hz), 5.55 (s,2H), 2.12 (s,3H). LC/MS (M+1, retention time): 439.0, 4.13 min.

### Example 2

(a) Synthesis of tert-butyl 2-({[(2Z)-5-methyl-3-(1-naphthylmethyl)-1,3-thiazol-2(3H)-ylidene]amino}-carbonyl)benzoate
   To a solution of 5-methyl-3-(1-naphthylmethyl)-1,3-thiazol-2(3H)-imine (139 mg, 0.546 mmol) and 2-(tert-butoxycarbonyl)benzoic acid (133 mg, 0.600 mmol) in N,N-dimethylformamide (7 ml) were added 1-ethyl-3-(3'-dimethylaminopropyl)carbodiimide monohydrochloride (126 mg, 0.658 mmol), hydroxybenzotriazole (88.9 mg, 0.658 mmol) and triethylamine (0.15 ml, 1.08 mmol), and the resulting mixture was stirred at 30°C for 1 hour. Then, the reaction solution was poured into water and extracted with ethyl acetate. The organic layer was washed with water and a saturated aqueous sodium chloride solution and then dried over anhydrous sodium sulfate. The solvent was distilled off under reduced pressure and the resulting residue was purified by a silica gel column chromatography (eluent: hexane/ethyl acetate = 8/2). The thus obtained solid was washed with diethyl ether/hexane to obtain the title compound (143 mg, 57.1%).; ¹H-NMR(DMSO-d₆) 8.22 (m,1H), 7.90-7.99 (m,2H), 7.79-7.85 (m,1H), 7.40-7.58 (m,6H), 7.28 (d, 1H, J=7.2Hz), 5.88 (s,2H), 2.24 (s,3H), 1.43 (s, 9H). LC/MS (M+1, retention time): 459.4, 4.25 min.
(b) Synthesis of 2-({[(2Z)-5-methyl-3-(1-naphthylmethyl)-1,3-thiazol-2(3H)-ylidene]amino}carbonyl)benzoic acid
   To a solution of tert-butyl 2-({[(2Z)-5-methyl-3-(1-naphthylmethyl)-1,3-thiazol-2(3H)-ylidene]amino}carbonyl)-benzoate (131 mg, 0.286 mmol) in acetic acid (5 ml) was added 4M hydrochloric acid/1,4-dioxane (2.0 ml, 8.00 mmol), and the resulting mixture was stirred at 50°C for 2 hours. After completion of the reaction, the solvent was distilled off under reduced pressure and to the resulting residue was added diethyl ether. The solid formed was collected by filtration to obtain the title compound (75.5 mg, 65.6%) . ; ¹H-NMR(DMSO-d₆) 8.15-8.25 (m,1H), 7.85-8.05 (m,3H), 7.45-7.60 (m,6H), 7.38 (d,1H,J=7.2Hz), 7.10 (br,1H), 2.21 (brs,3H). LC/MS (M+1, retention time): 403.4, 4.05 min.

### Example 3

(a) Synthesis of methyl 2-({[(2Z)-5-methyl-3-(1-naphthylmethyl)-1,3-thiazol-2(3H)-ylidene]amino}methyl)benzoate
   The title compound was synthesized by reacting 5-methyl-3-(1-naphthylmethyl)-1,3-thiazol-2(3H)-imine with methyl 2-(bromomethyl)benzoate according to the method described in Reference Example 2.; LC/MS (M+1, retention time): 403.1, 3.76 min.
(b) Synthesis of 2-({[(2Z)-5-methyl-3-(1-naphthylmethyl)-1,3-thiazol-2(3H)-ylidene]amino}methyl)benzoic acid
   The title compound was synthesized by hydrolyzing methyl 2-({[(2Z)-5-methyl-3-(1-naphthylmethyl)-1,3-thiazol-2(3H)-ylidene]amino}methyl)benzoate according to the method described in Example 1, (b).; LC/MS (M+1, retention time): 389.4, 3.49 min.

### Reference Example 4

### Synthesis of 2,2,2-trifluoro-N-(4-methyl-1,3-thiazol-2-yl)acetamide

The title compound was synthesized by reacting 2-amino-5-methylthiazole with trifluoroacetic anhydride according to the method described in Reference Example 1.; LC/MS (M+1, retention time): 210.7, 2.89 min.

### Reference Example 5

### Synthesis of 2,2,2-trifluoro-N-[(2Z)-4-methyl-3-(1-naphthylmethyl)-1,3-thiazol-2(3H)-ylidene]acetamide

The title compound was synthesized by reacting 2,2,2-trifluoro-N-(4-methyl-1,3-thiazol-2-yl)acetamide with 1-(chloromethyl)-naphthalene according to the method described in Reference Example 2.; LC/MS (M+1, retention time): 351.3, 4.15 min.

### Reference Example 6

### Synthesis of 4-methyl-3-(1-naphthylmethyl)-1,3-thiazol-2(3H)-imine

The title compound was synthesized by hydrolyzing 2,2,2-trifluoro-N-[(2Z)-4-methyl-3-(1-naphthylmethyl)-1,3-thiazol-2(3H)-ylidene]acetamide according to the method described in Reference Example 3.; LC/MS (M+1, retention time): 255.2, 2.37 min.

### Example 4

(a) Synthesis of methyl 2-({[(2Z)-4-methyl-3-(1-naphthylmethyl)-1,3-thiazol-2(3H)-ylidene]amino}-sulfonyl)benzoate
   The title compound was synthesized by reacting 4-methyl-3-(1-naphthylmethyl)-1,3-thiazol-2(3H)-imine with methyl 2-(chlorosulfonyl)benzoate according to the method described in Reference Example 1, (a).; LC/MS (M+1, retention time): 453.0, 2.97 min.
(b) Synthesis of 2-({[(2Z)-4-methyl-3-(1-naphthylmethyl)-1,3-thiazol-2(3H)-ylidene]amino}sulfonyl)benzoic acid
   The title compound was synthesized by hydrolyzing methyl 2-({[(2Z)-4-methyl-3-(1-naphthylmethyl)-1,3-thiazol-2(3H)-ylidene]amino}sulfonyl)benzoate according to the method described in Reference Example 1, (b).; ¹H-NMR(DMSO-d₆) 8.08-8.20 (m,1H), 7.96-8.01 (m,1H), 7.87 (d, 1H, J=7.7Hz), 7.74 (d, 1H, J=7.7Hz), 7.36-7.63 (m,6H), 6.74 (s,1H), 6.61 (d,1H,J=7.7Hz), 2.05 (s,3H). LC/MS (M+1, retention time): 439.0, 3.89 min.

### Reference Example 7

### Synthesis of N-(5-ethyl-1,3-thiazol-2-yl)-2,2,2-trifluoroacetamide

Bromine (1.71 ml, 33.3 mmol) was added dropwise to a solution (30 ml) of butyraldehyde (2.40 g, 33.3 mmol) in acetic acid at room temperature. After the resulting mixture was stirred at room temperature for 2 hours, thiourea (2.78 g, 36.6 mmol) was added to the reaction solution and the resulting mixture was refluxed for 2 hours. After completion of the reaction, the precipitate was filtered and the solvent of the filtrate was distilled off under reduced pressure. The resulting residue was dissolved in chloroform and the resulting solution was washed with a saturated aqueous sodium hydrogencarbonate solution and a saturated aqueous sodium chloride solution and then dried over anhydrous sodium sulfate. The solvent was distilled off under reduced pressure to obtain 3.15 g of a liquid substance containing 2-amino-5-ethylthiazole. Subsequently, the title compound was synthesized by reacting the residue obtained by the above procedure with trifluoroacetic anhydride according to the method described in Reference Example 1.; LC/MS (M+1, retention time): 225.2, 3.35 min.

### Reference Example 8

### Synthesis of N-[(2Z)-5-ethyl-3-(1-naphthylmethyl)-1,3-thiazol-2(3H)-ylidene]-2,2,2-trifluoroacetamide

The title compound was synthesized by reacting N-(5-ethyl-1,3-thiazol-2-yl)-2,2,2-trifluoroacetamide with 1-(chloromethyl)-naphthalene according to the method described in Reference Example 2.; ¹H-NMR(CDCl₃) 8.05-8.13 (m,1H), 7.85-7.95 (m,2H), 7.45-7.58 (m,4H), 6.61 (br,1H), 5.82 (s,2H), 2.53-2.62 (m,2H), 1.17 (d, 1H, J=7 . 5Hz) . LC/MS (M+1, retention time): 365.3, 4.75 min.

### Reference Example 9

### Synthesis of 5-ethyl-3-(1-naphthylmethyl)-1,3-thiazol-2(3H)-imine

The title compound was synthesized by hydrolyzing N-[(2Z)-5-ethyl-3-(1-naphthylmethyl)-1,3-thiazol-2(3H)-ylidene]-2,2,2-trifluoroacetamide according to the method described in Reference Example 3.; ¹H-NMR(CDCl₃) 8.05-8.10 (m,1H), 7.80-7.90 (m,2H), 7.37-7.60 (m,4H), 5.88 (br, 1H), 5.27 (s,2H), 2.29 (dq,2H,J=1.1,7.3Hz), 1.19 (d,1H,J=7.3Hz). LC/MS (M+1, retention time): 269.2, 3.05 min.

### Example 5

(a) Synthesis of methyl 2-({[(2Z)-5-ethyl-3-(1-naphthylmethyl)-1,3-thiazol-2(3H)-ylidene]amino}sulfonyl)benzoate
   The title compound was synthesized by reacting 5-ethyl-3-(1-naphthylmethyl)-1,3-thiazol-2(3H)-imine with methyl 2-(chlorosulfonyl)benzoate according to the method described in Example 1, (a).; ¹H-NMR(CDCl₃) 8.11-8.18 (m, 1H), 7.83-7.92 (m,3H), 7.27-7.60 (m,7H), 6.26 (br, 1H), 5.51 (s,2H), 3.89 (s,3H), 2.39-2.48 (m,2H), 1.10 (d,1H,J=7.4Hz). LC/MS (M+1, retention time): 467.3, 4.37 min.
(b) Synthesis of 2-({[(2Z)-5-ethyl-3-(1-naphthylmethyl)-1,3-thiazol-2(3H)-ylidene]amino}sulfonyl)benzoic acid
   The title compound was synthesized by hydrolyzing methyl 2-({[(2Z)-5-ethyl-3-(1-naphthylmethyl)-1,3-thiazol-2(3H)-ylidene]amino}sulfonyl)benzoate according to the method described in Example 1, (b).; LC/MS (M+1, retention time): 453.0, 4.04 min.

### Example 6

(a) Synthesis of tert-butyl 2-({[(2Z)-5-ethyl-3-(1-naphthylmethyl)-1,3-thiazol-2(3H)-ylidene]amino}carbonyl)benzoate
   The title compound was synthesized by condensing 5-ethyl-3-(1-naphthylmethyl)-1,3-thiazol-2(3H)-imine with 2-(tert-butoxycarbonyl)benzoic acid according to the method described in Example 2, (a).; ¹H-NMR(CDCl₃) 8.05-8.09 (m,1H), 7.94-7.99 (m,1H), 7.86-7.94 (m,2H), 7.60-7.67 (m,1H), 7.30-7.55 (m,6H), 6.46 (brs,1H), 5.80 (s,2H), 2.55 (m,2H), 1.55 (s,9H), 1.73 (t,3H,J=7.5Hz). LC/MS (M+1, retention time): 473.1, 4.98 min.
(b) Synthesis of 2-({[(2Z)-5-ethyl-3-(1-naphthylmethyl)-1,3-thiazol-2(3H)-ylidene]amino}carbonyl)benzoic acid
   The title compound was synthesized by removing the tert-butyl group of tert-butyl 2-({[(2Z)-5-ethyl-3-(1-naphthylmethyl)-1,3-thiazol-2(3H)-ylidene]amino}-carbonyl)benzoate according to the method described in Example 2, (b).; ¹H-NMR(CDCl₃) 8.18 (d,1H, J=7.7Hz), 8.03 (br,1H), 7.80-7.95 (m,4H), 7.40-7.67 (m,7H), 6.56 (s,1H), 6.20 (s,2H), 2.58 (q,2H,J=7.2Hz), 1.75 (t, 3H, J=7.2Hz).

### Reference Example 10

### Synthesis of 2,2,2-trifluoro-N-[(2Z)-5-isopropyl-3-(1-naphthylmethyl)-1,3-thiazol-2(3H)-ylidene]acetamide

The title compound was synthesized according to the methods described in Reference Examples 7 and 8. That is, isovaleraldehyde was reacted with bromine and thiourea according to the method described in Reference Example 7 to synthesize 4-isopropyl-2-trifluoroacetylaminothiazole. Subsequently, this compound was reacted with 1-(chloromethyl)-naphthalene according to the method described in Reference Example 8 to synthesize the title compound.; ¹H-NMR(CDCl₃) 8.11-8.18 (m,1H), 7.85-7.95 (m,2H), 7.45-7.58 (m,4H), 6.61 (br,1H), 5.82 (s,2H), 2.85-2.95 (m,1H), 1.19 (d,1H,J=7.0Hz). LC/MS (M+1, retention time): 379.0, 4.91 min.

### Reference Example 11

### Synthesis of 5-isopropyl-3-(1-naphthylmethyl)-1,3-thiazol-2(3H)-imine

The title compound was synthesized by hydrolyzing 2,2,2-trifluoro-N-[(2Z)-5-isopropyl-3-(1-naphthylmethyl)-1,3-thiazol-2(3H)-ylidene]acetamide according to the method described in Reference Example 3.; LC/MS (M+1, retention time): 283.2, 1.66 min.

### Example 7

(a) Synthesis of methyl 2-({[(2Z)-5-isopropyl-3-(1-naphthylmethyl)-1,3-thiazol-2(3H)-ylidene]amino}-sulfonyl)benzoate
   The title compound was synthesized by reacting 5-isopropyl-3-(1-naphthylmethyl)-1,3-thiazol-2(3H)-imine with methyl 2-(chlorosulfonyl)benzoate according to the method described in Example 1, (a).; ¹H-NMR(CDCl₃) 8.11-8.16 (m,1H), 7.84-7.93 (m,3H), 7.28-7.60 (m,7H), 6.26 (d,1H,J=1.1Hz), 5.51 (s,2H), 3.88 (s,3H), 2.71-2.82 (m,1H), 1.12 (d,1H,J=6.8Hz).
(b) Synthesis of 2-({[(2Z)-5-isopropyl-3-(1-naphthylmethyl)-1,3-thiazol-2(3H)-ylidene]amino}sulfonyl)benzoic acid
   The title compound was synthesized by hydrolyzing methyl 2-({[(2Z)-5-isopropyl-3-(1-naphthylmethyl)-1,3-thiazol-2(3H)-ylidene]amino}sulfonyl)benzoate according to the method described in Example 1, (b).; ¹H-NMR(CDCl₃) 8.17-8.22 (m,1H), 7.96-8.01 (m,1H), 7.81-7.90 (m,3H), 7.61-7.68 (m,2H),7.35-7.55 (m,4H), 6.24 (d,1H,J=1.1Hz), 5.46(s,2H),2.69-2.80 (m,1H), 1.09 (d,1H,J=6.8Hz). LC/MS (M+1, retention time): 467.3, 4.17 min.

### Reference Example 12

### Synthesis of 2-(4-hydroxybutyl)-1H-isoindole-1, 3 (2H) -dione

Phthalic anhydride (3.30 g, 22.4 mmol) was added to a solution of 4-amino-1-butanol (2.00 g, 22.4 mmol) in toluene (100 ml) and the resulting mixture was refluxed for 3 hours. After completion of the reaction, a saturated aqueous sodium chloride solution was added thereto, followed by extraction with ethyl acetate. The organic layer was washed successively with a 2M aqueous hydrochloric acid solution, a saturated aqueous sodium chloride solution, a saturated aqueous sodium hydrogencarbonate solution and a saturated aqueous sodium chloride solution and then dried over anhydrous sodium sulfate. The solvent was distilled off under reduced pressure and the resulting residue was purified by a silica gel column chromatography (eluent: hexane/ethyl acetate = 4/6) to obtain the title compound (2.00 g, 40.7%).; ¹H-NMR (CDCl₃) 7.80-7.88 (m,2H), 7.69-7.76 (m,2H), 3.66-3.78 (m,4H), 1.57-1.84 (m,4H). LC/MS (M+1, retention time): 220.2, 2.59 min.

### Reference Example 13

### Synthesis of 4-(1,3-dioxo-1,3-dihydro-2H-isoindol-2-yl)butanal

A solution (5 ml) of dimethyl sulfoxide (2.59 ml, 36.5 mmol) in dichloromethane was added dropwise to a solution (5 ml) of oxalyl chloride (1.59 ml, 18.2 mmol) in dichloromethane at -78°C, followed by adding dropwise thereto a solution (10 ml) of 2-(4-hydroxybutyl)-1H-isoindole-1,3(2H)-dione (2.00 g, 9.12 mmol) in dichloromethane. After stirring for 20 minutes, a solution (10 ml) of triethylamine (5.1 ml, 36.5 mmol) in dichloromethane was added thereto and the reaction temperature was raised to 0°C, followed by stirring for another 30 minutes. After completion of the reaction, a saturated aqueous sodium chloride solution was added thereto, followed by extraction with ethyl acetate. The organic layer was washed with water and a saturated aqueous sodium chloride solution and then dried over anhydrous sodium sulfate. The solvent was distilled off under reduced pressure to obtain the title compound (1.94 g, 97.9%).; ¹H-NMR(CDCl₃) 9.78 (brs,1H) 7.80-7.89 (m,2H),7.70-7.76 (m,2H), 3.75 (t,1H, J=6.8Hz), 2.55 (t, 1H, J=6. 8Hz), 2.02 (tt, 2H, J=6. 8, 6. 8Hz) .

### Reference Example 14

### Synthesis of 2-bromo-4-(1,3-dioxo-1,3-dihydro-2H-isoindol-2-yl)butanal

To a solution (5 ml) of 4-(1,3-dioxo-1,3-dihydro-2H-isoindol-2-yl)butanal (873 mg, 4.02 mmol) in acetonitrile was added 5,5-dibromobarbituric acid (689 mg, 2.41 mmol), and the resulting mixture was refluxed for 2 hours. After completion of the reaction, water was added thereto, followed by extraction with ethyl acetate. The organic layer was washed with a saturated aqueous sodium chloride solution and then dried over anhydrous sodium sulfate. The solvent was distilled off under reduced pressure and the resulting residue was purified by a silica gel column chromatography (eluent: hexane/ethyl acetate = 1/1) to obtain the title compound (1.16 g, 97%).; ¹H-NMR(CDCl₃) 9.48 (s,1H), 7.80-7.89 (m,2H), 7.70-7.77 (m,2H), 4.32-4.39 (m,1H), 3.82-3.95 (m,2H), 2.50-2.62 (m,1H), 2.21-2.30 (m, 1H) .

### Reference Example 15

### Synthesis of 2-[2-(2-amino-1,3-thiazol-5-yl)ethyl]-1H-isoindole-1,3 (2H)-dione

Thiourea (617 mg, 8.10 mmol) was added to a solution (30 ml) of 2-bromo-4-(1,3-dioxo-1,3-dihydro-2H-isoindol-2-yl)butanal (2.00 g, 6.75 mmol) in acetic acid and the resulting mixture was refluxed for 1 hour. After completion of the reaction, the solvent was distilled off under reduced pressure and chloroform and an aqueous sodium hydrogencarbonate solution were added to the residue, followed by extraction with chloroform. The organic layer was washed with a saturated aqueous sodium hydrogencarbonate solution and a saturated aqueous sodium chloride solution and then dried over anhydrous sodium sulfate. The solvent was distilled off under reduced pressure to obtain the title compound (a yellow solid, 1.38 g, 74.7%).; ¹H-NMR(CDCl₃) 7.80-8.00 (m,2H),7.26-7.76 (m,2H), 6.76 (s,1H), 4.93 (br,2H), 3.89 (t,2H,J=7.2Hz), 3.06 (t,2H,J=7.2Hz). LC/MS (M+1, retention time): 274.3, 2.53 min.

### Reference Example 16

### Synthesis of tert-butyl [2-(2-amino-1,3-thiazol-5-yl)ethyl]carbamate

Hydrazine hydrate (0.34 ml, 6.84 mmol) was added to a solution of 2-[2-(2-amino-1,3-thiazol-5-yl)ethyl]-1H-isoindole-1,3(2H)-dione (748 mg, 2.74 mmol) in a mixture of ethanol (35 ml) and methanol (5 ml), and the resulting mixture was stirred at 120°C for 2 hours. Subsequently, hydrazine hydrate (0.15 ml, 3.03 mmol) was added thereto, followed by stirring at 120°C for another 2 hours, whereupon a white precipitate was formed. After completion of the reaction, the reaction mixture was cooled to 0°C and the precipitate was collected by filtration. The solvent of the filtrate was distilled off under reduced pressure. The resulting residue was dissolved in a mixed solvent of N,N-dimethylformamide (10 ml), tetrahydrofuran (10 ml) and water (5 ml), and a solution (10 ml) of di-tert-butyl carbonate (89.5 mg, 0.410 mmol) in tetrahydrofuran was added dropwise thereto at room temperature, followed by stirring at 60°C for 2 hours. After completion of the reaction, the tetrahydrofuran was distilled off under reduced pressure and a saturated aqueous sodium chloride solution was added to the residue, followed by extraction with ethyl acetate. The extract solution was washed with a saturated aqueous sodium hydrogencarbonate solution and a saturated aqueous sodium chloride solution and then dried over anhydrous sodium sulfate. The solvent was distilled off under reduced pressure and the resulting residue was purified by a silica gel column chromatography (eluent: chloroform/methanol = 20/1) to obtain the title compound (613 mg, 92.1% from the two steps).; ¹H-NMR(CDCl₃) 6.76 (s, 1H), 5.02 (br,2H), 4.81 (br, 1H), 3. 30 (dd, 1H, J=6.5, 6.5Hz), 2.82 (t, 1H, J=6.5Hz). LC/MS (M+1, retention time): 244.3, 2.78 min.

### Reference Example 17

### Synthesis of tert-butyl (2-{2-[(trifluoroacetyl)-amino]-1,3-thiazol-5-yl}ethyl)carbamate

The title compound was synthesized by reacting tert-butyl [2-(2-amino-1,3-thiazol-5-yl)ethyl]carbamate with trifluoroacetic anhydride according to the method described in Reference Example 1.; ¹H-NMR(DMSO-d₆) 13.84 (m, 1H), 7.38 (s, 1H), 7.00 (t, 1H, J=6.3Hz), 3.14 (dd,2H,J=6.3,6.3Hz), 2.79 (t,2H,J=6.3Hz). LC/MS (M+1, retention time): 340.1, 3.12 min.

### Reference Example 18

### Synthesis of tert-butyl (2-{(2Z)-3-(1-naphthylmethyl)-2-[(trifluoroacetyl)imino]-2,3-dihydro-1,3-thiazol-5-yl}ethyl)carbamate

The title compound was synthesized by reacting tert-butyl (2-{2-[(trifluoroacetyl)amino]-1,3-thiazol-5-yl}ethyl)carbamate with 1-(chloromethyl)-naphthalene according to the method described in Reference Example 2.; ¹H-NMR(CDCl₃) 8.05-8.10 (m, 1H), 7.83-7.95 (m, 2H), 7.50-7.58 (m, 4H), 6.70 (s, 1H), 5.81 (s, 1H), 4.64 (br, 1H), 3.25 (dd, 2H, J=6.5, 6.5Hz), 2.74 (t,2H,J=6.5Hz). LC/MS (M+1, retention time): 480.1, 4.30 min.

### Reference Example 19

### Synthesis of tert-butyl {2-[2-imino-3-(1-naphthyl-methyl)-2,3-dihydro-1,3-thiazol-5-yl]ethyl}carbamate

The title compound was synthesized by hydrolyzing tert-butyl (2-{(2Z)-3-(1-naphthylmethyl)-2-[(trifluoroacetyl)imino]-2,3-dihydro-1,3-thiazol-5-yl}ethyl)carbamate according to the method described in Reference Example 3.; LC/MS (M+1, retention time): 384.1, 3.64 min.

### Example 8

(a) Synthesis of methyl 2-({[(2Z)-5-{2-[(tert-butoxycarbonyl)amino]ethyl}-3-(1-naphthylmethyl)-1,3-thiazol-2(3H)-ylidene]amino}sulfonyl)benzoate
   The title compound was synthesized by reacting tert-butyl {2-[2-imino-3-(1-naphthylmethyl)-2,3-dihydro-1,3-thiazol-5-yl]ethyl}carbamate with methyl 2-(chlorosulfonyl)benzoate according to the method described in Example 1, (a).; ¹H-NMR(DMSO-d₆) 7.84-8.01 (m,4H), 7.38-7.73 (m,5H), 7.22-7.34 (m,2H), 7.09 (s,1H), 6.96 (t,1H,J=6.4Hz), 3.68 (s,3H), 3.01-3.10 (m,2H), 2.64 (t,2H,J=6.4Hz). LC/MS (M+1, retention time): 582.1, 4.85 min.
(b) Synthesis of 2-({[(2Z)-5-{2-[(tert-butoxycarbonyl)amino]ethyl}-3-(1-naphthylmethyl)-1,3-thiazol-2(3H)-ylidene]amino}sulfonyl)benzoic acid
   The title compound was synthesized by hydrolyzing methyl 2-({[(2Z)-5-{2-[(tert-butoxycarbonyl)amino]ethyl}-3-(1-naphthylmethyl)-1,3-thiazol-2(3H)-ylidene]amino}-sulfonyl)benzoate according to the method described in Example 1, (b).; ¹H-NMR(DMSO-d₆) 7.80-8.05 (m,4H), 7.25-7.62 (m,7H), 7.02 (s,1H), 6.95 (br,1H), 5.57 (s,2H), 3.04 (br,2H), 2.61 (br,2H). LC/MS (M+1, retention time): 467.3, 4.17 min.
(c) Synthesis of methyl 2-({[(2Z)-5-(2-aminoethyl)-3-(1-naphthylmethyl)-1,3-thiazol-2(3H)-ylidene]amino}sulfonyl)benzoate trifluoroacetate
   Trifluoroacetic acid (4 ml) was added to a solution of methyl 2-({[(2Z)-5-{2-[(tert-butoxycarbonyl)amino]ethyl}-3-(1-naphthylmethyl)-1,3-thiazol-2(3H)-ylidene]amino}-sulfonyl)benzoate (295 mg, 0.508 mmol) in dichloromethane (8 ml), and the resulting mixture was stirred overnight at room temperature. After completion of the reaction, the solvent was distilled off under reduced pressure to obtain the title compound (298 mg, quantitative).; LC/MS (M+1, retention time): 482.1, 3.23 min.
(d) Synthesis of methyl 2-({[(2Z)-5-{2-[(ethoxycarbonyl)amino]ethyl}-3-(1-naphthylmethyl)-1,3-thiazol-2(3H)-ylidene]amino}sulfonyl)benzoate
   Ethyl chloroformate (0.028 ml, 0.291 mmol) and triethylamine (0.101 ml, 0.729 mmol) were added to a solution (4 ml) of methyl 2-({[(2Z)-5-(2-aminoethyl)-3-(1-naphthylmethyl)-1,3-thiazol-2(3H)-ylidene]amino}sulfonyl)benzoate trifluoroacetate (148 mg, 0.243 mmol) in dichloromethane at 0°C and stirred at room temperature for 1 hour. After completion of the reaction, the reaction solution was cooled to 0°C and a saturated aqueous sodium chloride solution was added thereto, followed by extraction with ethyl acetate. The organic layer was washed successively with a 2M aqueous hydrochloric acid solution, a saturated aqueous sodium chloride solution, a saturated aqueous sodium hydrogencarbonate solution and a saturated aqueous sodium chloride solution and then dried over anhydrous sodium sulfate. The solvent was distilled off under reduced pressure and the resulting residue was purified by a silica gel column chromatography (eluent: hexane/ethyl acetate = 5/5) to obtain the title compound (58.9 mg, 43.8%).; ¹H-NMR(CDCl₃) 8.10-8.15 (m,1H), 7.80-7.88 (m,3H), 7.24-7.62 (m,7H), 6.37 (s,1H), 5.50 (s,2H), 4.90 (br,1H), 4.01 (q,2H,J=7.2Hz), 3.88 (s,3H), 3.24 (q,2H,J=6.6Hz), 2.61 (t,2H,J=6.6Hz), 1.17 (t,3H,J=7.2Hz). LC/MS (M+1, retention time): 554.1, 3.58 min.
(e) Synthesis of 2-({[(2Z)-5-{2-[(ethoxycarbonyl)amino]ethyl}-3-(1-naphthylmethyl)-1,3-thiazol-2(3H-ylidene]amino}sulfonyl)benzoic acid
   The title compound was synthesized by hydrolyzing methyl 2-({[(2Z)-5-{2-[(ethoxycarbonyl)amino]ethyl}-3-(1-naphthylmethyl)-1,3-thiazol-2(3H)-ylidene]amino}sulfonyl)benzoate according to the method described in Example 1, (b).; LC/MS (M+1, retention time): 540.1, 3.85 min.
(f) Synthesis of methyl 2-({[(2Z) -3- (1-naphthylmethyl)-5-{2-[(tetrahydro-2H-pyran-4-ylcarbonyl)amino]ethyl}- 1,3-thiazol-2(3H)-ylidene]amino}sulfonyl)benzoate
   The title compound was synthesized by condensing methyl 2-({[(2Z)-5-(2-aminoethyl)-3-(1-naphthylmethyl)-1,3-thiazol-2(3H)-ylidene]amino}sulfonyl)benzoate trifluoroacetate with tetrahydropyran-4-yl-carboxylic acid according to the method described in Example 2, (a).; LC/MS (M+1, retention time): 594.4, 3.80 min.
(g) Synthesis of 2-({[(2Z)-3-(1-naphthylmethyl)-5-{2-[(tetrahydro-2H-pyran-4-ylcarbonyl)amino]ethyl}-1,3-thiazol-2(3H)-ylidene]amino}sulfonyl)benzoic acid
   The title compound was synthesized by hydrolyzing methyl 2-({[(2Z)-3-(1-naphthylmethyl)-5-{2-[(tetrahydro-2H-pyran-4-ylcarbonyl)amino]ethyl}-1,3-thiazol-2(3H)-ylidene]amino}sulfonyl)benzoate according to the method described in Example 1, (b).;
   ¹H-NMR(DMSO-d₆) 13.20 (brs, 1H), 7.84-8.03 (m,4H), 7.25-7.68 (m,7H), 7.02 (s,1H), 5.56 (s,2H), 3.72-3.80 (m,2H), 3.10-3.24 (m,6H), 2.65 (br,2H), 2.19 (br,1H), 1.35-1.50 (m,4H). LC/MS (M+1, retention time): 580.1, 3.06 min.
(h) Synthesis of methyl 2-({[(2Z)-5-[2-(isonicotinoyl-amino)ethyl]-3-(1-naphthylmethyl)-1,3-thiazol-2(3H)-ylidene]amino}sulfonyl)benzoate
   The title compound was synthesized by condensing methyl 2-({[(2Z)-5-(2-aminoethyl)-3-(1-naphthylmethyl)-1,3-thiazol-2(3H)-ylidene]amino}sulfonyl)benzoate trifluoroacetate with 4-picolinic acid according to the method described in Example 2, (a).; LC/MS (M+1, retention time): 587.2, 3.43 min.
   (i) Synthesis of 2-({[(2Z)-5-[2-(isonicotinoylamino)ethyl]-3-(1-naphthylmethyl)-1,3-thiazol-2(3H)-ylidene]-amino}sulfonyl)benzoic acid
      The title compound was synthesized by hydrolyzing methyl 2-({[(2Z)-5-[2-(isonicotinoylamino)ethyl]-3-(1-naphthylmethyl)-1,3-thiazol-2(3H)-ylidene]amino}sulfonyl)benzoate according to the method described in Example 1, (b).; LC/MS (M+1, retention time): 573.1, 3.22 min.

### Reference Example 20

### Synthesis of 2-[2-(1,3-dioxolan-2-yl)ethyl]-1H-isoindole-1,3(2H)-dione

A solution of 2-(2-bromomethyl)-1,3-dioxolane (90%, 2.00 g, 11.1 mmol) and potassium phthalimide (2.05 g, 11.1 mmol) in N,N-dimethylformamide (20 ml) was stirred at 100°C for 1.5 hours. After completion of the reaction, a saturated aqueous sodium chloride solution was added thereto, followed by extraction with ethyl acetate. The organic layer was washed successively with water, a saturated aqueous sodium hydrogencarbonate solution and a saturated aqueous sodium chloride solution and then dried over anhydrous sodium sulfate. The solvent was distilled off under reduced pressure and the thus obtained solid was washed with a hexane-diethyl ether mixed solvent to obtain the title compound (2.06 g, 75.7%).; ¹H-NMR(CDCl₃) 7.80-7.85 (m,2H), 7.67-7.75 (m,2H), 4.96 (t,2H,J=4.4Hz), 3.78-4.00 (m,6H), 2.08 (dt,2H,J=4.4,6.9Hz).

### Reference Example 21

### Synthesis of 3-(1,3-dioxo-1,3-dihydro-2H-isoindol-2-yl)propanal

In a mixed solvent of tetrahydrofuran (12 ml) and acetic acid (4 ml) was dissolved 2-[2-(1,3-dioxolan-2-yl)ethyl]-1H-isoindole-1,3(2H)-dione (728 mg, 2.94 mmol), followed by adding thereto a 2M aqueous hydrochloric acid solution (5 ml) at room temperature, and the resulting mixture was stirred at 50°C for 2 hours. After completion of the reaction, the reaction solution was extracted with ethyl acetate. The organic layer was washed successively with water, a saturated aqueous sodium hydrogencarbonate solution and a saturated aqueous sodium chloride solution and then dried over anhydrous sodium sulfate. The solvent was distilled off under reduced pressure and the thus obtained solid was washed with a hexane-diethyl ether mixed solvent to obtain the title compound (546 mg, 91.4%).; ¹H-NMR(DMSO-d₆) 9.65 (br, 1H), 7.80-7.88 (m,4H), 3.84 (t,2H,J=4.4Hz), 2.79 (dt,2H,J=1.5,6.9Hz).

### Reference Example 22

### Synthesis of 2-[(2-amino-1,3-thiazol-5-yl)methyl]-1H-isoindole-1,3(2H)-dione

Bromine (0.22 ml, 4.29 mmol) was added dropwise to a solution (30 ml) of 3-(1,3-dioxo-1,3-dihydro-2H-isoindol-2-yl)propanal (866 mg, 4.36 mmol) in acetic acid at room temperature. After stirring at room temperature for 2 hours, the reaction solution changed in color from brown to colorless. Thiourea (373 mg, 4.90 mmol) was added to the reaction solution and the resulting mixture was refluxed for 2 hours. After completion of the reaction, the solvent was distilled off under reduced pressure and the resulting residue was washed with methanol-diethyl ether and then dried. The solvent was distilled off under reduced pressure to obtain the title compound (616 mg, 55.8%).; LC/MS (M+1, retention time): 260.0, 3.76 min.

### Reference Example 23

### Synthesis of tert-butyl [(2-amino-1,3-thiazol-5-yl)methyl]carbamate

According to the method described in Reference Example 16, the title compound was synthesized by removing the phthaloyl-protecting group of 2-[(2-amino-1,3-thiazol-5-yl)methyl]-1H-isoindole-1,3(2H)-dione and protecting the resulting amine with a tert-butylcarbonyl group.; ¹H-NMR(CDCl₃) 6.89 (brs,1H), 4.84 (brs,1H), 4.28 (d,2H,J=5.5Hz), 1.46 (s,9H).

### Reference Example 24

### Synthesis of tert-butyl ({2-[(trifluoroacetyl)amino]-1,3-thiazol-5-yl}methyl)carbamate

The title compound was synthesized by reacting tert-butyl [(2-amino-1,3-thiazol-5-yl)methyl]carbamate with trifluoroacetic anhydride according to the method described in Reference Example 1.; LC/MS (M+1, retention time): 326.1, 3.17 min.

### Reference Example 25

### Synthesis of tert-butyl ({(2Z)-3-(1-naphthylmethyl)-2-[(trifluoroacetyl)imino]-2,3-dihydro-1,3-thiazol-5-yl}methyl)carbamate

The title compound was synthesized by reacting tert-butyl ({2-[(trifluoroacetyl)amino]-1,3-thiazol-5-yl}methyl)carbamate with 1-(chloromethyl)-naphthalene according to the method described in Reference Example 2.; ¹H-NMR(CDCl₃) 8.06 (br,1H), 7.80-7.96 (m,2H), 7.45-7.58 (m,4H), 6.82 (s,1H), 5.82 (s,2H), 4.87 (br,1H), 4.18 (br,2H), 1.39 (s,9H). LC/MS (M+1, retention time): 466.1, 4.72 min.

### Reference Example 26

### Synthesis of tert-butyl {[2-imino-3-(1-naphthylmethyl)-2,3-dihydro-1,3-thiazol-5-yl]methyl}carbamate

The title compound was synthesized by hydrolyzing tert-butyl ({(2Z)-3-(1-naphthylmethyl)-2-[(trifluoroacetyl)imino]-2,3-dihydro-1,3-thiazol-5-yl}methyl)carbamate according to the method described in Reference Example 3.; LC/MS (M+1, retention time): 370.1, 3.19 min.

### Example 9

(a) Synthesis of methyl 2-({[(2Z)-5-{[(tert-butoxycarbonyl)amino]methyl}-3-(1-naphthylmethyl)-1,3-thiazol-2(3H)-ylidene]amino}sulfonyl)benzoate
   The title compound was synthesized by reacting tert-butyl {[2-imino-3-(1-naphthylmethyl)-2,3-dihydro-1,3-thiazol-5-yl]methyl}carbamate with methyl 2-(chlorosulfonyl)benzoate according to the method described in Example 1, (a).; ¹H-NMR(CDCl₃) 8.14-8.17 (m,1H), 7.83-7.90 (m,3H), 7.37-7.63 (m,6H), 7.23-7.30 (m, 1H), 6.46 (brs, 1H), 5.50 (s,2H), 4.82 (brs, 1H), 4.04 (br,2H), 3.89 (s,3H), 1.37 (s,9H).
(b) Synthesis of 2-({[(2Z)-5-{[(tert-butoxycarbonyl)-amino]methyl}-3-(1-naphthylmethyl)-1,3-thiazol-2(3H)-ylidene]amino}sulfonyl)benzoic acid
   The title compound was synthesized by hydrolyzing methyl 2-({[(2Z)-5-{[(tert-butoxycarbonyl)amino]methyl}-3-(1-naphthylmethyl)-1,3-thiazol-2(3H)-ylidene]amino}sulfonyl)benzoate according to the method described in Example 1, (b).; ¹H-NMR(DMSO-d₆) 13.25 (brs,1H), 7.78-8.02 (m,4H), 7.25-7.70 (m,7H), 7.08 (s,1H), 5.57 (s,2H), 3.97 (br,2H), 1.34 (s,9H). LC/MS (M+1, retention time): 554.1, 4.08 min.
(c) Synthesis of methyl 2-({[(2Z)-5-(aminomethyl)-3-(1-naphthylmethyl)-1,3-thiazol-2(3H)-ylidene]amino}sulfonyl)benzoate
   Trifluoroacetic acid (1.5 ml) was added to a solution of methyl 2-({[(2Z) 5-{[(tert-butoxycarbonyl)amino]methyl}-3-(1-naphthylmethyl)-1,3-thiazol-2(3H)-ylidene]amino}sulfonyl)benzoate (86.0 mg, 0.151 mmol) in dichloromethane (3 ml), and the resulting mixture was stirred overnight at room temperature. After completion of the reaction, the solvent was distilled off under reduced pressure and the resulting residue was dissolved in chloroform. The organic layer was washed with a saturated aqueous sodium hydrogencarbonate solution and then a saturated aqueous sodium chloride solution, and dried over anhydrous sodium sulfate. The solvent was distilled off under reduced pressure to obtain the title compound (72.0 mg, 82.0%).; LC/MS (M+1, retention time): 468.1, 0.76 min.
(d) Synthesis of methyl 2-({[(2Z)-3-(1-naphthylmethyl)-5-{[(pyridin-4-ylmethyl)(trifluoroacetyl)amino]methyl}-1,3-thiazol-2(3H)-ylidene]amino}sulfonyl)benzoate
   A solution of methyl 2-({[(2Z)-5-(aminomethyl)-3-(1-naphthylmethyl)-1,3-thiazol-2(3H)-ylidene]amino}sulfonyl)benzoate (86.0 mg, 0.151 mmol) and 4-formylpyridine (18.0 mg, 0.168 mmol) in 1,2-dichloroethane (4 ml) was stirred at room temperature for 1 hour. Then, sodium triacetoxy borohydride (51.2 mg, 0.230 mmol) and acetic acid (0.02 ml) were added to the solution at room temperature and stirred at room temperature for 3 hours. A saturated aqueous sodium hydrogencarbonate solution was poured into the reaction mixture, followed by extraction with chloroform. The organic layer was washed with a saturated aqueous sodium chloride solution and then dried over anhydrous sodium sulfate. The solvent was distilled off under reduced pressure and the resulting residue was purified by a silica gel column chromatography (eluent: chloroform/methanol = 30/1). The solvent was distilled off under reduced pressure and the resulting residue (56.4 mg) was dissolved in dichloromethane. To the resulting solution were added trifluoroacetic anhydride (0.06 ml, 0.425 mmol), triethylamine (0.10 ml, 0.720 mmol) and a catalytic amount of 4-dimethylaminopyridine, followed by stirring at room temperature for 1 hour. After completion of the reaction, the reaction solution was cooled to 0°C and a saturated aqueous sodium chloride solution was added thereto, followed by extraction with ethyl acetate. The organic layer was washed with a saturated aqueous sodium hydrogencarbonate solution and then a saturated aqueous sodium chloride solution, and dried over anhydrous sodium sulfate. The solvent was distilled off under reduced pressure and the resulting residue was purified by a silica gel column chromatography (eluent: hexane/ethyl acetate = 5/5) to obtain the title compound (35.8 mg, 36.2%).; LC/MS (M+1, retention time): 655.2, 2.98 min.
(e) Synthesis of 2-({[(2Z)-3-(1-naphthylmethyl)-5-{[(pyridin-4-ylmethyl)amino]methyl}-1,3-thiazol-2(3H)-ylidene]amino}sulfonyl)benzoic acid
   The title compound was synthesized by hydrolyzing methyl 2-({[(2Z)-3-(1-naphthylmethyl)-5-([(pyridin-4-ylmethyl)(trifluoroacetyl)amino]methyl}-1,3-thiazol-2(3H)-ylidene]amino}sulfonyl)benzoate according to the method described in Example 1, (b).; ¹H-NMR(DMSO-d₆) 8.43 (d,2H,J=5.3Hz), 7.85-8.00 (m,4H), 7.25-7.70 (m,7H), 7.20 (d,2H,J=5.3Hz), 7.07 (s,1H), 5.57 (s,2H), 3.50-3.66 (m,4H). LC/MS (M+1, retention time): 545.1, 2.45 min.

### Reference Example 27

### Synthesis of tert-butyl 4-(2-hydroxyethyl)-piperidine-1-carboxylate

A solution (20 ml) of di-tert-butyl carbonate (8.95 g, 41.0 mmol) in tetrahydrofuran was added dropwise to a solution (80 ml) of 4-piperidineethanol (5.30 g, 41.0 mmol) in tetrahydrofuran at room temperature and stirred overnight. After completion of the reaction, the solvent was distilled off under reduced pressure and the residue was dissolved in ethyl acetate. The resulting solution was washed successively with a 1M aqueous hydrochloric acid solution, a saturated aqueous sodium chloride solution, a saturated aqueous sodium hydrogencarbonate solution and a saturated aqueous sodium chloride solution and dried over anhydrous sodium sulfate. The solvent was distilled off under reduced pressure and the resulting residue was purified by a silica gel column chromatography (eluent: chloroform/methanol = 98/2) to obtain the title compound (9.67 g, quantitative).; ¹H-NMR(CDCl₃) 4.18 (br,2H), 3.66-3.74 (m,2H), 2.69 (br,2H), 1.68 (br,2H), 1.45-1.57 (m,2H).

### Reference Example 28

### Synthesis of tert-butyl 4-(2-oxoethyl)piperidine-1-carboxylate

The title compound was synthesized by oxidizing tert-butyl 4-(2-hydroxyethyl)piperidine-1-carboxylate according to the method described in Reference Example 13.; ¹H-NMR(CDCl₃) 9.78 (t,1H,J=1.7Hz), 4.09 (br,2H), 2.74 (br,2H), 2.39 (dd,2H,J=6.8,1.7Hz), 1.98-2.13 (m,1H), 1.68 (br,2H), 1.10-1.27 (m,2H).

### Reference Example 29

### Synthesis of tert-butyl 4-(2-amino-1,3-thiazol-5-yl)piperidine-1-carboxylate

The title compound was synthesized according to the methods described in Reference Examples 14 and 15. That is, tert-butyl 4-(2-oxoethyl)piperidine-1-carboxylate was reacted with 5,5-dibromobarbituric acid according to the method described in Reference Example 14, and then the resulting α-bromoaldehyde was reacted with thiourea according to the method described in Reference Example 15, to synthesize the title compound.; ¹H-NMR(CDCl₃) 6.75 (s, 1H), 5.05 (br,2H), 4.14 (br,2H), 2.72-2.88 (m,3H), 1.90 (br,2H), 1.40-1.60 (m,2H).

### Reference Example 30

### Synthesis of tert-butyl 4-{2-[(trifluoroacetyl)amino]-1,3-thiazol-5-yl}piperidine-1-carboxylate

The title compound was synthesized by reacting tert-butyl 4-(2-amino-1,3-thiazol-5-yl)piperidine-1-carboxylate with trifluoroacetic anhydride according to the method described in Reference Example 1.; ¹H-NMR(CDCl₃) 7.05 (s,1H), 4.23 (br,2H), 2.71-3.00 (m,3H), 2.00 (br,2H), 1.54-1.71 (m,2H). LC/MS (M+1, retention time): 380.1, 3.67 min.

### Reference Example 31

### Synthesis of tert-butyl 4-{(2Z)-3-(1-naphthylmethyl)-2-[(trifluoroacetyl)imino]-2,3-dihydro-1,3-thiazol-5-yl}piperidine-1-carboxylate

The title compound was synthesized by reacting tert-butyl 4-{2-[(trifluoroacetyl)amino]-1,3-thiazol-5-yl}piperidine-1-carboxylate with 1-(chloromethyl)-naphthalene according to the method described in Reference Example 2.; ¹H-NMR(CDCl₃) 8.05-8.12 (m,1H), 7.80-7.95 (m,2H), 7.40-7.60 (m,4H), 6.61 (s,1H), 5.81 (s,2H), 4.13 (br,2H), 2.69 (br,3H), 1.80 (br,2H), 1.36-1.52 (m,2H). LC/MS (M+1, retention time): 520.2, 5.43 min.

### Reference Example 32

### Synthesis of tert-butyl 4-[2-imino-3-(1-naphthylmethyl)-2,3-dihydro-1,3-thiazol-5-yl]piperidine-1-carboxylate

The title compound was synthesized by hydrolyzing tert-butyl 4-{(2Z)-3-(1-naphthylmethyl)-2-[(trifluoroacetyl)imino]-2,3-dihydro-1,3-thiazol-5-yl}piperidine-1-carboxylate according to the method described in Reference Example 3.; ¹H-NMR(CDCl₃) 8.03 (d,1H,J=7.3Hz), 7.83-7.90 (m,2H), 7.31-7.60 (m,4H), 5.90 (s,1H), 5.31 (s,2H), 4.08 (br,2H), 2.66 (br,2H), 2.39 (br,1H), 1.68 (br,2H), 1.20-1.46 (m,2H). LC/MS (M+1, retention time): 424.1, 3.03 min.

### Example 10

(a) Synthesis of tert-butyl 4-[(2Z)-2-({[2-(methoxycarbonyl)phenyl]sulfonyl}imino)-3-(1-naphthylmethyl)-2,3-dihydro-1,3-thiazol-5-yl]piperidine-1-carboxylate
   The title compound was synthesized by reacting tert-butyl 4-[2-imino-3-(1-naphthylmethyl)-2,3-dihydro-1,3-thiazol-5-yl]piperidine-1-carboxylate with methyl 2-(chlorosulfonyl)benzoate according to the method described in Example 1, (a).; ¹H-NMR(CDCl₃) 8.14 (d,1H,J=7.0Hz), 7.88 (d,2H,J=8.4Hz), 7.26-7.61 (m,8H), 6.27 (s,1H), 5.51 (s,2H), 4.08 (br,2H), 3.89 (s,3H), 2.49-2.80 (m,3H), 1.70-1.76 (m,2H), 1.30-1.48 (m,2H). LC/MS (M+1, retention time): 622.4, 4.19 min.
(b) Synthesis of 2-({[(2Z)-5-[1-(tert-butoxycarbonyl)-piperidin-4-yl]-3-(1-naphthylmethyl)-1,3-thiazol-2(3H)-ylidene]amino}sulfonyl)benzoic acid
   The title compound was synthesized by hydrolyzing tert-butyl 4-[(2Z)-2-({[2-(methoxycarbonyl)phenyl]sulfonyl}imino)-3-(1-naphthylmethyl)-2,3-dihydro-1,3-thiazol-5-yl]piperidine-1-carboxylate according to the method described in Example 1, (b).; ¹H-NMR(CDCl₃) 8.16-8.21 (m,1H), 7.80-8.00 (m,4H),7.63-7.67 (m,2H), 4.09 (br,2H), 2.45-2.74 (m,3H), 1.60-1.75 (m,2H), 1.20-1.41 (m,2H). LC/MS (M+1, retention time): 608.1, 3.87 min.
(c) Synthesis of 2-({[(2Z)-3-(1-naphthylmethyl)-5-piperidin-4-yl-1,3-thiazol-2(3H)-ylidene]amino}-sulfonyl)benzoic acid hydrochloride
   To a solution of 2-({[(2Z)-5-[1-(tert-butoxycarbonyl)piperidin-4-yl]-3-(1-naphthylmethyl)-1,3-thiazol-2(3H)-ylidene]amino}sulfonyl)benzoic acid (28.0 mg, 0.0461 mmol) in acetic acid (3 ml) was added 4M hydrochloric acid/1,4-dioxane (0.50 ml), and the resulting mixture was stirred overnight at room temperature. After completion of the reaction, the solvent was distilled off under reduced pressure to obtain the title compound (28.8 mg, quantitative).; LC/MS (M+1, retention time): 508.1, 2.87 min.

### Reference Example 33

### Synthesis of tert-butyl (2-amino-4,5,6,7-tetrahydro-1,3-benzothiazol-6-yl)carbamate

A solution (5 ml) of di-tert-butyl carbonate (262 mg, 1.20 mmol) in tetrahydrofuran was added dropwise to a solution of 4,5,6,7-tetrahydro-1,3-benzothiazole-2,6-diamine (203 mg, 1.20 mmol) known in literature (J. Med. Chem., 30, 494. (1987)) in N,N-dimethylformamide (5 ml) at room temperature and stirred at 50°C for 1 hour. After completion of the reaction, a saturated aqueous sodium chloride solution was added thereto, followed by extraction with ethyl acetate. The organic layer was washed successively with a saturated aqueous sodium hydrogencarbonate solution, water and a saturated aqueous sodium chloride solution and dried over anhydrous sodium sulfate. The solvent was distilled off under reduced pressure and the resulting residue was purified by a silica gel column chromatography (eluent: chloroform/methanol = 20/1) to obtain the title compound (9.67 mg, quantitative).; LC/MS (M+1, retention time): 270.3, 2.96 min.

### Reference Example 34

### Synthesis of tert-butyl {2-[(trifluoroacetyl)amino]-4,5,6,7-tetrahydro-1,3-benzothiazol-6-yl}carbamate

The title compound was synthesized by reacting tert-butyl (2-amino-4,5,6,7-tetrahydro-1,3-benzothiazol-6-yl)carbamate with trifluoroacetic anhydride according to the method described in Reference Example 1.; LC/MS (M+1, retention time): 366.1, 3.53 min.

### Reference Example 35

### Synthesis of tert-butyl {(2Z)-3-(1-naphthylmethyl)-2-[(trifluoroacetyl)imino]-2,3,4,5,6,7-hexahydro-1,3-benzothiazol-6-yl}carbamate

The title compound was synthesized by reacting tert-butyl {2-[(trifluoroacetyl)amino]-4,5,6,7-tetrahydro-1,3-benzothiazol-6-yl}carbamate with 1-(chloromethyl)-naphthalene according to the method described in Reference Example 2.; LC/MS (M+1, retention time): 506.2, 4.27 min.

### Reference Example 36

### Synthesis of tert-butyl [2-imino-3-(1-naphthylmethyl)-2,3,4,5,6,7-hexahydro-1,3-benzothiazol-6-yl]carbamate

The title compound was synthesized by hydrolyzing tert-butyl {(2Z)-3-(1-naphthylmethyl)-2-[(trifluoroacetyl)-imino]-2,3,4,5,6,7-hexahydro-1,3-benzothiazol-6-yl}carbamate according to the method described in Reference Example 3.; LC/MS (M+1, retention time): 410.1, 3.65 min.

### Example 11

(a) Synthesis of methyl 2-({[(2Z)-6-[(tert-butoxycarbonyl)-amino]-3-(1-naphthylmethyl)-4,5,6,7-tetrahydro-1,3-benzothiazol-2(3H)-ylidene]amino}sulfonyl)benzoate
   The title compound was synthesized by reacting tert-butyl [2-imino-3-(1-naphthylmethyl)-2,3,4,5,6,7-hexahydro-1,3-benzothiazol-6-yl]carbamate with methyl 2-(chlorosulfonyl)benzoate according to the method described in Example 1, (a).; LC/MS (M+1, retention time): 608.2, 4.84 min.
(b) Synthesis of 2-({[(2Z)-6-[(tert-butoxycarbonyl)amino]-3-(1-naphthylmethyl)-4,5,6,7-tetrahydro-1,3-benzothiazol-2 (3H)-ylidene]amino}sulfonyl)benzoic acid
   The title compound was synthesized by hydrolyzing methyl 2-({[(2Z)-6-[(tert-butoxycarbonyl)amino]-3-(1-naphthylmethyl)-4,5,6,7-tetrahydro-1,3-benzothiazol-2(3H)-ylidene]amino}sulfonyl)benzoate according to the method described in Example 1, (b).; ¹H-NMR(DMSO-d₆) 13.23 (brs, 1H), 8.09 (br, 1H), 7.98 (br, 1H), 7.87 (d,1H,J=7.9Hz), 7.75 (d,1H,J=7.9Hz), 7.37-7.62 (m,6H), 7.05 (br,1H), 6.73 (br,1H), 5.65 (s,2H), 3.65 (br,1H), 2.77 (br,1H), 2.34 (br,3H), 1.75 (br,1H), 1.58 (br,1H). LC/MS (M+1, retention time): 594.2, 4.59 min.

### Reference Example 37

(a) Synthesis of tert-butyl 2-[(1,3-thiazol-2-ylamino)carbonyl]benzoate
   The title compound was synthesized by condensing 2-aminothiazole with 2-(tert-butoxycarbonyl)benzoic acid according to the method described in Example 2, (a).; LC/MS (M+1, retention time): 305.4, 3.76 min.

### Example 12

Synthesis of tert-butyl 2-({[(2Z)-3-(1-naphthylmethyl)-1,3-thiazol-2(3H)-ylidene]amino}carbonyl)benzoate

The title compound was synthesized by reacting tert-butyl 2-[(1,3-thiazol-2-ylamino)carbonyl]benzoate with 1-(chloromethyl)-naphthalene according to the method described in Reference Example 2.; LC/MS (M+1, retention time): 445.4, 4.73 min.

### Example 13

Synthesis of 2-({[(2Z)-3-(1-naphthylmethyl)-1,3-thiazol-2(3H)-ylidene]amino}carbonyl)benzoic acid

The title compound was synthesized by removing the tert-butyl group of tert-butyl 2-({[(2Z)-3-(1-naphthylmethyl)-1,3-thiazol-2(3H)-ylidene]amino}carbonyl)benzoate according to the method described in Reference Example 2, (b).; ¹H-NMR(DMSO-d₆) 8.15-8.25 (m,1H), 7.80-8.02 (m,3H), 7.45-7.60 (m,5H), 7.40-7.43 (m,1H), 7.30 (d,1H,J=7.2Hz), 7.07-7.10 (m,1H), 5.94 (s,2H). LC/MS (M+1,retention time): 389.4, 4.00 min.

### Test Example

### Inhibitory effect on chymase (in vitro test)

### [Test method]

To 100 µl of a buffer solution A (50 mM Tris-hydrochloric acid (pH = 8.0), 2M NaCl) were added 50 ng of chymase derived from human skin (Elastin Products Co.), a fluorescent synthetic substrate 0.1 mM succinyl-alanylprolyl-phenylalanine-methylcoumalylamide (Peptide Laboratories Co., Ltd.) and a test substance dissolved in dimethyl sulfoxide (DMSO), and the resulting mixture was incubated at 37°C for 2 hours. Then, the intensity of fluorescence was measured at an excitation wavelength of 355 nm and a measuring wavelength of 460 nm by the use of a fluorescence plate reader (Fluoroscan (Dainippon Pharmaceutical Co., Ltd.)). A fluorescence intensity value obtained without adding the test substance was taken as 100% and a concentration at which a fluorescence intensity value calculated from a regression line became 50% was taken as IC₅₀.

### [Test result]

The chymase inhibitory effect IC₅₀ of the compound of Example 1 was 2.1 nM.

### INDUSTRIAL APPLICABILITY

The compounds of the present invention have inhibitory effect on chymase and are useful as therapeutic agents for diseases whose pathosis are considered improvable by this effect, such as the following diseases in which mast cell activation, angiotensin II, endothelin or the like is involved: hypertension, cardiac failure, ischemic peripheral circulatory disturbance, myocardial ischemia, venous malfunction, cardiac failure advance after myocardiac infarction, diabetic nephropathy, nephritis, arteriosclerosis, hyperaldosteronism, scleroderma, glomerulosclerosis, renal failure, central nervous system diseases, Alzheimer's disease, hypomnesia, depression, sensory functional disorders including amnesia and senile dementia, anxiety and tension, unpleasant mental condition, glaucoma, ocular hypertension, restenosis after PTCA, asthma, rhinitis, COPD, allergic diseases such as atopic dermatitis, or the like.

## Claims

1. A compound represented by formula (1): 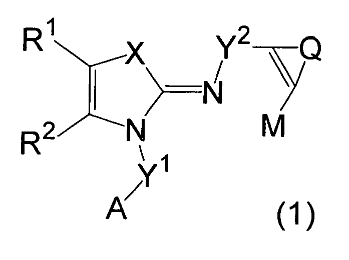 wherein X is a sulfur atom or an oxygen atom;
R¹ and R² are independently a group represented by the formula: -Y³-Z, or R¹ and R², when taken together, represent a substituted or unsubstituted alkylene group (the -CH₂- groups of the alkylene group may be replaced by one or more substituents which may be the same or different and are selected from groups represented by the formulas: -O-, -S(O)ₙ-, -N(R¹¹)- and -C (=O) -) ;
Y³ is a single bond or a substituted or unsubstituted alkylene group (the -CH₂- groups of the alkylene group may be replaced by one or more substituents which may be the same or different and are selected from groups represented by the formulas: -O-, -S(O)ₙ-, -N(R¹¹)- and -C(=O)-, substituted or unsubstituted benzene rings, and substituted or unsubstituted cycloalkane rings);
Y¹ and Y² are independently a substituted or unsubstituted alkylene group (the -CH₂- groups of the alkylene group may be replaced by one or more substituents which may be the same or different and are selected from groups represented by the formulas: -O-, -S(O)ₙ-, -N (R¹¹) - and -C (=O) -, substituted or unsubstituted benzene rings, and substituted or unsubstituted cycloalkane rings, provided that the end of the alkylene group directly bonded to each nitrogen atom in formula (1) is not a group represented by the formula: -N (R¹¹)-);
the -CH₂- groups in a cycloalkane ring in the case of the cycloalkane ring being present in any of Y¹, Y² and Y³ may be replaced by one or more substituents which may be the same or different and are selected from groups represented by the formulas: -O-, -S(O)ₙ-, - N (R¹¹) - and -C (=O) -;
any adjacent two carbon atoms of an alkylene group may form a double bond or a triple bond in the case of the alkylene group being present as any of Y¹, Y² and Y³ or in the case of R¹ and R² being taken together to represent the alkylene group;
Z is a saturated or unsaturated monocyclic hydrocarbon ring group, a saturated or unsaturated polycyclic hydrocarbon ring group, a saturated or unsaturated monocyclic heterocyclic group, or a saturated or unsaturated polycyclic heterocyclic group (these groups may be unsubstituted or substituted) or is a hydrogen atom, a halogen atom, a nitro group, a cyano group, a substituted or unsubstituted alkyl group, a substituted or unsubstituted alkenyl group, a substituted or unsubstituted alkynyl group, a substituted or unsubstituted acyl group, or a group represented by the formula: -OR²¹, -N (R²²) R²³, -C (=O) OR²¹, -S(O)ₙR²⁴, -C (=O) R²⁵, -C (=O) N (R²²) R²³, -N (R²⁶) C (=O) R²⁵,-S(O)₂N(R²²)R²³, -N (R²⁶) S (O)ₙR²⁴ or -N (R²⁶) C (=O) OR²¹;
M is a group represented by the formula: - C (=O) OR³¹, -S(O)ₙOR³¹, -C (=O) N (R³²) R³³, -S (O) nN (R³²) R³³ or - N(R³⁴)S(O)ₙR³⁵, a tetrazol-5-yl group, a 1,2,4-triazol-3-yl group, a 1,2,4-triazol-5-yl group, an imidazol-2-yl group or an imidazol-4-yl group;
Q is taken together with the group represented by the formula: -C=C- to which Q is bonded, to represent a benzene ring or a 5- or 6-membered heteroaromatic ring (these rings may be unsubstituted or substituted);
A is a saturated or unsaturated monocyclic hydrocarbon ring group, a saturated or unsaturated polycyclic hydrocarbon ring group, a saturated or unsaturated monocyclic heterocyclic group, or a saturated or unsaturated polycyclic heterocyclic group (these groups may be unsubstituted or substituted);
R¹¹, R²¹, R²², R²³, R²⁴, R²⁵, R²⁶, R³¹, R³², R³³, R³⁴ and R³⁵, which may be the same or different, are independently as follows (when any of them is present as two or more substituents, these substituents are independently as follows): a hydrogen atom, a substituted or unsubstituted alkyl group, a substituted or unsubstituted alkenyl group, a substituted or unsubstituted cycloalkyl group or a substituted or unsubstituted aralkyl group, each of a combination of R²² and R²³ and a combination of R³² and R³³ being able to be taken together with the nitrogen atom to which the combination is bonded, to represent a saturated 3- to 8-membered cyclic amino group which may contain other heteroatoms in the ring (said cyclic amino group may be unsubstituted or substituted), provided that each of R²⁴ and R³⁵ is not a hydrogen atom when the number of oxygen atoms (n) on the sulfur atom bonded to R²⁴ or R³⁵, respectively, is 1 or 2; and
n is 0, 1 or 2 (when n is present as two or more suffixes, these suffixes are independently 0, 1 or 2),
a prodrug of said compound, or a pharmaceutically acceptable salt of said compound or prodrug.

2. A compound, a prodrug thereof or a pharmaceutically acceptable salt of the compound or prodrug according to claim 1, wherein X is a sulfur atom.

3. A compound, a prodrug thereof or a pharmaceutically acceptable salt of the compound or prodrug according to claim 1 or 2, wherein Y² is a group represented by the formula: -S(O)₂-, -C(=O)- or -CH₂-.

4. A compound, a prodrug thereof or a pharmaceutically acceptable salt of the compound or prodrug according to claim 1, 2 or 3, wherein Q is taken together with the group represented by the formula: -C=C- to which Q is bonded, to represent an unsubstituted or substituted o-phenylene group.

5. A compound, a prodrug thereof or a pharmaceutically acceptable salt of the compound or prodrug according to any one of claims 1 to 4, wherein M is a group represented by the formula: -C(=O)OR³¹.

6. A compound, a prodrug thereof or a pharmaceutically acceptable salt of the compound or prodrug according to any one of claims 1 to 5, wherein Y¹ is a substituted or unsubstituted C₁₋₆alkylene group.

7. A compound, a prodrug thereof or a pharmaceutically acceptable salt of the compound or prodrug according to any one of claims 1 to 6, wherein R² is hydroxyl group, a cyano group, a halogen atom or an unsubstituted C₁₋₆alkyl group.

8. A compound, a prodrug thereof or a pharmaceutically acceptable salt of the compound or prodrug according to any one of claims 1 to 7, wherein A is a 1-naphthyl group or a 2-naphthyl group.

9. A compound, a prodrug thereof or a pharmaceutically acceptable salt of the compound or prodrug according to any one of claims 1 to 8, wherein in one or both of R¹ and R², Z is a saturated or unsaturated monocyclic hydrocarbon ring group, a saturated or unsaturated polycyclic hydrocarbon ring group, a saturated or unsaturated monocyclic heterocyclic group, or a saturated or unsaturated polycyclic heterocyclic group, and each of these groups is substituted by a group represented by the formula: - Y⁴-Z' in which
Y⁴ is a single bond or a substituted or unsubstituted alkylene group (the -CH₂- groups of the alkylene group may be replaced by one or more substituents which may be the same or different and are selected from groups represented by the formulas: -O-, -S (O)ₙ-, -N (R¹¹) - and -C(=O)-, substituted or unsubstituted benzene rings, and substituted or unsubstituted cycloalkane rings (the -CH₂- groups in the cycloalkane ring may be replaced by one or more substituents which may be the same or different and are selected from groups represented by the formulas: -O-, -S(O)ₙ-, -N (R¹¹) - and -C (=O) -), and any adjacent two carbon atoms of the alkylene group may form a double bond or a triple bond);
Z' is a saturated or unsaturated monocyclic hydrocarbon ring group, a saturated or unsaturated polycyclic hydrocarbon ring group, a saturated or unsaturated monocyclic heterocyclic group, or a saturated or unsaturated polycyclic heterocyclic group (each of these groups may be either unsubstituted or substituted by one or more substituents which may be the same or different and are selected from halogen atoms, nitro group, cyano group, alkyl groups, aralkyl groups, alkoxy groups and alkylenedioxy groups); and
R¹¹ and n are as defined above (when either of them is present as two or more substituents or suffixes, respectively, these substituents or suffixes are independently as defined above).

10. A compound, a prodrug thereof or a pharmaceutically acceptable salt of the compound or prodrug according to any one of claims 1 to 9, wherein Y¹, Y², Y³ and Y⁴ are independently a group represented by the formula : -(CH₂)ₚ- (CH₂)_{q}-, -(CH₂)ₚ-O- (CH₂)_{q}-, - (CH₂)ₚ-S (O)ₙ- (CH₂)_{q}-, (CH₂)ₚ-N (R¹¹)- (CH₂)_{q}-, - (CH₂)ₚ₋C (=O) N (R¹¹) - (CH₂)_{q}-, - (CH₂)ₚ-N (R¹¹) C (=O)- (CH₂)_{q}- (CH₂)ₚ₋C (=O) O- (CH₂)_{q}-, - (CH₂)ₚ-OC (=O) (CH₂)_{q}-, - (CH₂)ₚ-SO₂N (R¹¹) - (CH₂)_{q}-, - (CH₂)ₚ-N (R¹¹) SO₂- (CH₂)_{q}- or - (CH₂)ₚ-R¹²- (CH²)_{q}-, which may be substituted or unsubstituted and in which
each of p and q is such an integer that p + q is 0 to 6, -(CH₂)ₚ- may form a double bond or a triple bond between its adjacent carbon atoms in the case of p being 2 or more, and -(CH₂)_{q}- may form a double bond or a triple bond between its adjacent carbon atoms in the case of q being 2 or more; and
R¹² is a substituted or unsubstituted benzene ring, or a substituted or unsubstituted cycloalkane ring.

11. A pharmaceutical composition comprising a compound, a prodrug thereof or a pharmaceutically acceptable salt of the compound or prodrug according to any one of claims 1 to 10.

12. A chymase inhibitor comprising a compound, a prodrug thereof or a pharmaceutically acceptable salt of the compound or prodrug according to any one of claims 1 to 10.

13. A pharmaceutical composition for the treatment of hypertension, cardiac failure, ischemic peripheral circulatory disturbance, myocardial ischemia, venous malfunction, cardiac failure advance after myocardiac infarction, diabetic nephropathy, nephritis, arteriosclerosis, hyperaldosteronism, scleroderma, glomerulosclerosis, renal failure, central nervous system diseases, Alzheimer's disease, hypomnesia, depression, sensory functional disorders, anxiety, tension, unpleasant mental condition, glaucoma, ocular hypertension, restenosis after PTCA, asthma, rhinitis, COPD or allergic diseases, which comprises a compound, a prodrug thereof or a pharmaceutically acceptable salt of the compound or prodrug according to any one of claims 1 to 10.
